# EUROPEAN PATENT SPECIFICATION

(11) **EP 0 560 613 B1**
(45) Date of publication and mention of the grant of the patent: **25.11.1998**
(21) Application number: 93301872.3
(22) Date of filing: 11.03.1993
(51) Int. Cl.: C07D 477/00, A61K 31/40

(54) **Antimicrobial carbapenem derivatives, their preparation and their therapeutic use**
Antimikrobielle Carbapenem-Derivate, ihre Herstellung und ihre therapeutische Verwendung
Dérivés antimicrobiens du carbapenem, leur préparation et leur utilisation thérapeutique

(30) Priority: 11.03.1992 JP 52163/92; 14.09.1992 JP 244953/92; 16.09.1992 JP 246578/92
(43) Date of publication of application: 15.09.1993
(73) Proprietor: SANKYO COMPANY LIMITED, Chuo-ku, Tokyo 103 (JP)
(72) Inventor: Kawamoto, Isao, c/o Sankyo Company Limited, Tokyo 140 (JP); Endo, Rokuro, c/o Sankyo Company Limited, Tokyo 140 (JP); Miyauchi, Masao, c/o Sankyo Company Limited, Tokyo 140 (JP); Ishikawa, Katsuya, c/o Sankyo Company Limited, Tokyo 140 (JP); Watanabe, Katsuhiko, c/o Sankyo Company Limited, Tokyo 140 (JP); Yasuda, Hiroshi, c/o Sankyo Company Limited, Tokyo 140 (JP); Ohya, Satoshi, c/o Sankyo Company Limited, Tokyo 140 (JP); Utsui, Yukio, c/o Sankyo Company Limited, Tokyo 140 (JP)
(74) Representative: Gibson, Christian John Robert

(56) References cited:
- EP-A- 0 126 587
- EP-A- 0 243 686
- EP-A- 0 442 497
- EP-A- 0 443 883
- EP-A- 0 518 558

## Description

The present invention relates to a series of carbapenem derivatives which have excellent antimicrobial activities and which, by virtue of their enhanced resistance to inactivation by dehydropeptidase I, have improved value in the therapy and prophylaxis of infectious diseases. The invention also provides methods of preparing these compounds, as well as methods and compositions using them.

The carbapenem compounds are a well known series of compounds, related to the penicillins, which have been used or have been proposed for use as antibiotics. They have in common a basic structure which may be represented by the formula (A):

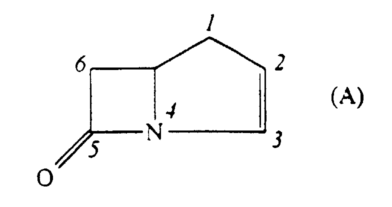

In this formula, we have indicated the numbering of those positions of importance to the carbapenem compounds, using the numbering scheme commonly used in the art and as employed in the nomenclature of the compounds of the present invention. In accordance with the recommendations of the International Union of Pure and Applied Chemistry (IUPAC), Commission on Nomenclature of Organic Chemistry, the compounds referred to herein are named semi-systematically, using the above carbapenem structure as the parent name.

Those carbapenem antibiotics having no substituent at the 1-position are potentially a very useful series of compounds which have extraordinarily potent antibacterial activity. Unfortunately, however, they are chemically unstable and, moreover, are sensitive to dehydropeptidase I in vivo. Dehydropeptidase I is an enzyme which hydrolyses the β-lactam ring in carbapenem antibiotics and which exists in mammalian tissue, for example in the renal cortex. It is responsible for the extensive metabolisation of many otherwise valuable β-lactam antibiotics in animals, including humans, thus greatly reducing their value. Despite these disadvantages, these carbapenem antibiotics are finding increasing use in the treatment of bacterial infections.

Metabolism of the antibiotic in vivo may be demonstrated by a low recovery of the compound itself (as opposed to its metabolic products) in the urine, and this has been demonstrated for thienamycin [H. Kropp et al., Antimicrob. Agents, Chemother., 22, 62 (1982); and S. R. Norrby et al., ibid., 23, 300 (1983)].

Although it has been found that carbapenem compounds having a substituent at the 1-position (commonly a 1-methyl group) do not have this susceptibility to dehydropeptidase I in vivo, many of the compounds of this type discovered to date lack sufficient activity. It is, therefore, considered highly desirable to find a carbapenem antibiotic which combines the good activity of thienamycin with a resistance to dehydropeptidase I in vivo.

Many carbapenem compounds are now known. Some are described, for example, in European Patent Publications No. 126 587, 243 686, 333 175, 442 497, 443 883, 508 682 and 518 558. European Patent Publication No. 333 175 discloses compounds in which a thio-pyrrolidinyl group and its ring carbon atom substituent are linked by an alkylene group, and thus differ from the compounds of the present invention in that there is no linking carbonyl group. The compounds disclosed in European Patent Publication 126 587, on the other hand, are carboxylic thio-pyrrolidinyl beta-lactam compounds. However, it is thought that the closest prior art to the compounds of the present invention is represented by European Patent Publications No. 508 682 and 518 558, both of which were published after the priority dates hereof. The present compounds have demonstrated significantly better activity than the other prior art compounds.

Thus, the compounds of the present invention are those compounds of formula (I) and pharmaceutically acceptable salts and esters thereof: 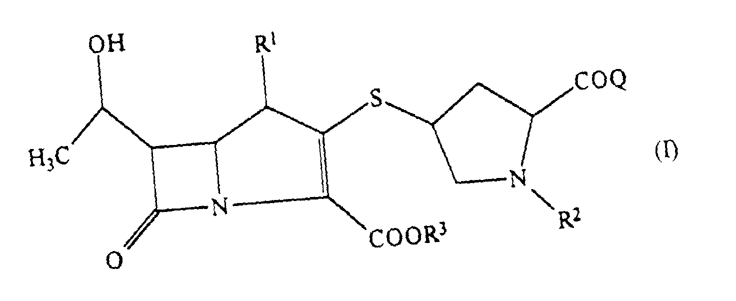 in which:
R¹ represents a hydrogen atom or a methyl group;
R² represents a hydrogen atom, an unsubstituted alkyl group having from 1 to 6 carbon atoms, a substituted alkyl group which has from 1 to 6 carbon atoms and which is substituted by at least one of substituents A, defined below, an alkenyl group having from 2 to 6 carbon atoms, an alkynyl group having from 2 to 6 carbon atoms, or a group of formula -C(=NH)R⁰,
   where R⁰ represents a hydrogen atom or an alkyl group having from 1 to 6 carbon atoms;
R³ represents a hydrogen atom, a negative ion or a carboxy-protecting group; and
Q represents a group of formula (Q-VII) or (Q-VIII): wherein: 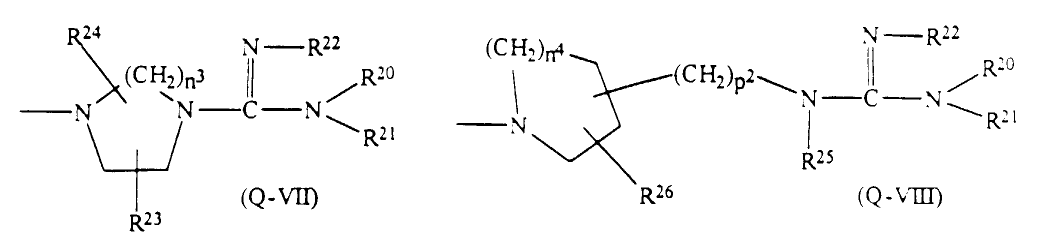
   R²⁰, R²¹ and R²² are independently selected from hydrogen atoms and unsubstituted alkyl groups having from 1 to 6 carbon atoms;
      or
   R²⁰ and R²¹ or R²⁰ and R²² together represent a group of formula -(CH₂)ₛ- (W)_{w}, -(CH₂)ₜ-, where s is 0, 1, 2 or 3, t is 0, 1, 2 or 3, W represents an oxygen or sulphur atom and w' is 0 or 1;
   R²³ and R²⁴ are independently selected from hydrogen atoms, halogen atoms, unsubstituted alkyl groups having from 1 to 6 carbon atoms, substituted alkyl groups which have from 1 to 6 carbon atoms and which are substituted by at least one of substituents C, defined below, hydroxy groups, carboxy groups, carbamoyl groups, amino groups, cyano groups and carbamoyloxy groups;
   n³ is 1, 2 or 3;
   R²⁵ represents a hydrogen atom, an unsubstituted alkyl group having from 1 to 6 carbon atoms, a substituted alkyl group which has from 1 to 6 carbon atoms and which is substituted by at least one of substituents C, defined below, an alkenyl group having from 2 to 6 carbon atoms, or an alkynyl group having from 2 to 6 carbon atoms;
   R²⁶ represents a hydrogen atom, a halogen atom, an unsubstituted alkyl group having from 1 to 6 carbon atoms, a substituted alkyl group which has from 1 to 6 carbon atoms and which is substituted by at least one of substituents C, defined below, a hydroxy group, a carboxy group, a carbamoyl group, an amino group, a cyano group or a carbamoyloxy group;
   n⁴ is 0, 1 or 2;
   p² is 0 or 1;
   substituents A are selected from: hydroxy groups, carboxy groups, carbamoyl groups, carbamoyloxy groups, cyano groups, halogen atoms, oxygen atoms (to form an oxo group), alkoxy groups having from 1 to 6 carbon atoms, amino groups, alkylamino groups having from 1 to 6 carbon atoms and dialkylamino groups in which each alkyl part has from 1 to 6 carbon atoms; and
   substituents C are selected from: hydroxy groups, carboxy groups, carbamoyl groups, cyano groups, halogen atoms, alkoxy groups having from 1 to 6 carbon atoms and amino groups.

The present invention also provides a pharmaceutical composition comprising a pharmaceutically acceptable carrier, diluent or adjuvant in admixture with an effective amount of an antibiotic, wherein the antibiotic is selected from compounds of formula (I) and pharmaceutically acceptable salts and esters thereof.

The present invention further provides the use of an antibiotic, wherein the antibiotic is selected from compounds of formula (I) and pharmaceutically acceptable salts and esters thereof, as a pharmaceutical.

The present invention also provides processes for preparing these compounds, which are described in greater detail hereafter.

In the compounds of the present invention, where R²⁰, R²¹ R²², R²³, R²⁴, R²⁵ or R²⁶ represents an alkyl group having from 1 to 6 carbon atoms, this may be a straight or branched chain group having from 1 to 6, preferably from 1 to 4, carbon atoms, and examples include the methyl, ethyl, propyl, isopropyl, butyl, isobutyl, sec-butyl, t-butyl, pentyl, isopentyl, neopentyl, 2-methylbutyl, 1-ethylpropyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, hexyl and isohexyl groups. Of these, we prefer those alkyl groups having from 1 to 4 carbon atoms, preferably the methyl, ethyl, propyl, isopropyl, butyl, isobutyl, sec-butyl and t-butyl groups, and most preferably the methyl and ethyl groups.

Where R⁰ or R² represents an alkyl group having from 1 to 6 carbon atoms, this may be a straight or branched chain group having from 1 to 6, preferably from 1 to 4, carbon atoms, and examples include the methyl, ethyl, propyl, isopropyl, butyl, isobutyl, sec-butyl, t-butyl, pentyl, isopentyl, neopentyl, 2-methylbutyl, 1-ethylpropyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, hexyl and isohexyl groups. Of these, we prefer those alkyl groups having from 1 to 3 carbon atoms, preferably the methyl, ethyl and propyl groups, and most preferably the methyl group.

Where R² or R²⁵ represents an alkenyl group, this may be a straight or branched chain group having from 2 to 6, preferably 3 or 4, carbon atoms, and examples include the vinyl, allyl, 2-methylallyl, 1-propenyl, isopropenyl, 1-butenyl, 2-butenyl, 3-butenyl, 1-pentenyl, 2-pentenyl, 3-pentenyl, 4-pentenyl and 3-hexenyl groups, of which the vinyl, allyl, 2-methylallyl, 1-propenyl, isopropenyl and butenyl groups are preferred, the allyl and 2-methylallyl groups being most preferred.

Where R² or R²⁵ represents an alkynyl group, this may be a straight or branched chain group having from 2 to 6, preferably 3 or 4, carbon atoms, and examples include the ethynyl, propargyl (2-propynyl), 1-propynyl, 2-methylpropargyl, 1-butynyl, 2-butynyl, 3-butynyl, 1-pentynyl, 2-pentynyl, 3-pentynyl, 4-pentynyl and 3-hexynyl groups, of which the propynyl and 2-methylpropargyl groups are preferred.

Where R² represents a substituted alkyl group, the alkyl part itself may be any of the alkyl groups exemplified above in relation to the unsubstituted alkyl groups, and the substituents are selected from the group consisting of substituents A, for example:
hydroxy groups, carboxy groups, carbamoyl groups, carbamoyloxy groups, cyano groups;
halogen atoms, such as the fluorine, chlorine, bromine or iodine atoms, preferably the fluorine, chlorine or bromine atoms;
oxygen atoms (to form an oxo group) ;
alkoxy groups having from 1 to 6 carbon atoms, which may be straight or branched chain alkoxy groups, and examples include the methoxy, ethoxy, propoxy, isopropoxy, butoxy, isobutoxy, sec-butoxy, t-butoxy, pentyloxy, neopentyloxy, isopentyloxy and hexyloxygroups, of which we prefer those alkoxy groups having from 1 to 3 carbon atoms, more preferably the methoxy, ethoxy or propoxy group;
amino groups;
alkylamino groups having from 1 to 6 carbon atoms, in which the alkyl part may be as exemplified above in relation to R² etc., and examples include the methylamino, ethylamino, propylamino, isopropylamino, butylamino, isobutylamino, sec-butylamino, t-butylamino, pentylamino, isopentylamino, neopentylamino, and hexylamino groups, of which we prefer the methylamino, ethylamino and propylamino groups; and
dialkylamino groups in which each alkyl part has from 1 to 6 carbon atoms and may be as exemplified above in relation to R² etc.; examples include the dimethylamino, diethylamino, dipropylamino, diisopropylamino, dibutylamino, dipentylamino, dihexylamino, methylethylamino and methylpropylamino, of which we prefer the dimethylamino, diethylamino and dipropylamino groups.

In the case of substituents C examples of the groups and atoms which may be included in these are as given in relation to the equivalent groups and atoms of substituents A.

Where R³ represents a carboxy-protecting group, this is preferably a group capable of forming an ester with a carboxylic acid. Examples of ester groups include:
alkyl groups having from 1 to 20 carbon atoms, more preferably from 1 to 6 carbon atoms, such as those exemplified in relation to substituents R²⁰ and higher alkyl groups as are well known in the art, such as the heptyl, octyl, nonyl, decyl, dodecyl, tridecyl, pentadecyl, octadecyl, nonadecyl and icosyl groups, but preferably groups having from 1 to 4 carbon atoms and most preferably the methyl, ethyl and t-butyl groups;
cycloalkyl groups having from 3 to 7 carbon atoms, for example the cyclopropyl, cyclobutyl, cyclopentyl, cyclohexyl and cycloheptyl groups;
aralkyl groups, in which the alkyl part has from 1 to 3 carbon atoms and the aryl part is a carbocyclic aromatic group having from 6 to 14 carbon atoms, which may be substituted or unsubstituted and, if substituted, has at least one of substituents H defined and exemplified below, although the unsubstituted groups are preferred; examples of such aralkyl groups include the benzyl, phenethyl, 1-phenylethyl, 3-phenylpropyl, 2-phenylpropyl, 1-naphthylmethyl, 2-naphthylmethyl, 2-(1-naphthyl)ethyl, 2-(2-naphthyl)ethyl, benzhydryl (i.e. diphenylmethyl), triphenylmethyl, bis(o-nitrophenyl)methyl, 9-anthrylmethyl, 2,4,6-trimethylbenzyl, 4-bromobenzyl, 2-nitrobenzyl, 4-nitrobenzyl, 3-nitrobenzyl, 4-methoxybenzyl and piperonyl groups, of which the benzyl, benzhydryl, 4-nitrobenzyl and 2-nitrobenzyl groups are preferred;
alkenyl groups having from 2 to 6 carbon atoms, and halogenated alkenyl groups having from 2 to 6 carbon atoms, such as the the vinyl, allyl, 2-methylallyl, 2-chloroallyl, 1-propenyl, isopropenyl, 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl and 5-hexenyl groups, of which the vinyl, allyl, 2-methylallyl, 1-propenyl, isopropenyl and butenyl groups are preferred, the allyl, 2-chloroallyl and 2-methylallyl groups being most preferred.
halogenated alkyl groups having from 1 to 6, preferably from 1 to 4, carbon atoms, in which the alkyl part is as defined and exemplified in relation to the alkyl groups above, and the halogen atom is chlorine, fluorine, bromine or iodine (preferably chlorine or bromine), such as the 2,2,2-trichloroethyl, 2-haloethyl (e.g. 2-chloroethyl, 2-fluoroethyl, 2-bromoethyl or 2-iodoethyl), 2,2-dibromoethyl and 2,2,2-tribromoethyl groups, preferably the 2,2,2-trichloroethyl, 2,2-dibromoethyl and 2,2,2-tribromoethyl groups;
substituted silylalkyl groups, in which the alkyl part is as defined and exemplified above, and the silyl group has up to 3 substituents selected from alkyl groups having from 1 to 6 carbon atoms and phenyl groups which are unsubstituted or have at least one substituent selected from substituents H defined and exemplified below, for example a 2-trimethylsilylethyl group;
phenyl groups, in which the phenyl group is unsubstituted or substituted, preferably with at least one alkyl group having from 1 to 4 carbon atoms or acylamino group, for example the phenyl, tolyl and benzamidophenyl groups;
phenacyl groups, which may be unsubstituted or have at least one of substituents H defined and exemplified below, for example the phenacyl group itself or the p-bromophenacyl group;
cyclic and acyclic terpenyl groups, for example the geranyl, neryl, linalyl, phytyl, menthyl (especially m- and p-menthyl), thujyl, caryl, pinanyl, bornyl, notcaryl, norpinanyl, norbornyl, menthenyl, camphenyl and norbornenyl groups;
alkoxymethyl groups, in which the alkoxy part has from 1 to 6, preferably from 1 to 4, carbon atoms and may itself be substituted by a single unsubstituted alkoxy group, such as the methoxymethyl, ethoxymethyl, propoxymethyl, isopropoxymethyl, butoxymethyl and methoxyethoxymethyl groups;
aliphatic acyloxyalkyl groups, in which the acyl group is preferably an alkanoyl group and is more preferably an alkanoyl group having from 2 to 6 carbon atoms, and the alkyl part has from 1 to 6, and preferably from 1 to 4, carbon atoms such as the acetoxymethyl, propionyloxymethyl, butyryloxymethyl, isobutyryloxymethyl, pivaloyloxymethyl, 1-pivaloyloxyethyl, 1-acetoxyethyl, 1-isobutyryloxyethyl, 1-pivaloyloxypropyl, 2-methyl-1-pivaloyloxypropyl, 2-pivaloyloxypropyl, 1-isobutyryloxyethyl, 1-isobutyryloxypropyl, 1-acetoxypropyl, 1-acetoxy-2-methylpropyl, 1-propionyloxyethyl, 1-propionyloxypropyl, 2-acetoxypropyl and 1-butyryloxyethyl groups, preferably a pivaloyloxymethyl, isobutyryloxymethyl, 1-isobutyryloxyethyl, acetoxymethyl or 1-acetoxyethyl group;
cycloalkyl-substituted aliphatic acyloxyalkyl groups, in which the acyl group is preferably an alkanoyl group and is more preferably an alkanoyl group having from 2 to 6 carbon atoms, the cycloalkyl substituent has from 3 to 7 carbon atoms, and the alkyl part has from 1 to 6, preferably from 1 to 4, carbon atoms, such as the (cyclohexylacetoxy)methyl, 1-(cyclohexylacetoxy)ethyl, 1-(cyclohexylacetoxy)propyl, 2-methyl-1-(cyclohexylacetoxy)propyl, (cyclopentylacetoxy)methyl, 1-(cyclopentylacetoxy)ethyl, 1-(cyclopentylacetoxy)propyl and 2-methyl-1-(cyclopentylacetoxy)propyl, groups;
alkoxycarbonyloxyalkyl groups, especially 1-(alkoxycarbonyloxy)ethyl groups, in which the alkoxy part has from 1 to 10, preferably from 1 to 6, and more preferably from 1 to 4, carbon atoms, and the alkyl part has from 1 to 6, preferably from 1 to 4, carbon atoms, such as the t-butoxycarbonyloxyethyl, 1-methoxycarbonyloxyethyl, 1-ethoxycarbonyloxyethyl, 1-propoxycarbonyloxyethyl, 1-isopropoxycarbonyloxyethyl, 1-butoxycarbonyloxyethyl, 1-isobutoxycarbonyloxyethyl, 1-sec-butoxycarbonyloxyethyl, 1-t-butoxycarbonyloxyethyl, 1-(1-ethylpropoxycarbonyloxy)ethyl and 1-(1,1-dipropylbutoxycarbonyloxy)ethyl groups, and other alkoxycarbonylalkyl groups, in which both the alkoxy and alkyl groups have from 1 to 6, preferably from 1 to 4, carbon atoms, such as the 2-methyl-1-(isopropoxycarbonyloxy)propyl, 2-(isopropoxycarbonyloxy)propyl, isopropoxycarbonyloxymethyl, t-butoxycarbonyloxymethyl, methoxycarbonyloxymethyl and ethoxycarbonyloxymethyl groups; of these, the t-butoxycarbonyloxyethyl, 1-methoxycarbonyloxyethyl, 1-ethoxycarbonyloxyethyl, 1-isopropoxycarbonyloxyethyl and 1-t-butoxycarbonyloxyethyl groups are preferred;
cycloalkylcarbonyloxyalkyl and cycloalkyloxycarbonyloxyalkyl groups, in which the cycloalkyl group has from 3 to 10, preferably from 3 to 7, carbon atoms, is mono- or poly- cyclic and is optionally substituted by at least one (and preferably only one) alkyl group having from 1 to 4 carbon atoms (e.g. selected from those alkyl groups exemplified above) and the alkyl part has from 1 to 6, more preferably from 1 to 4, carbon atoms (e.g. selected from those alkyl groups exemplified above) and is most preferably methyl, ethyl or propyl, for example the 1-methylcyclohexylcarbonyloxymethyl, 1-methylcyclohexyloxycarbonyloxymethyl, cyclopentyloxycarbonyloxymethyl, cyclopentylcarbonyloxymethyl, 1-cyclohexyloxycarbonyloxyethyl, 1-cyclohexylcarbonyloxyethyl, 1-cyclopentyloxycarbonyloxyethyl, 1-cyclopentylcarbonyloxyethyl, 1-cycloheptyloxycarbonyloxyethyl, 1-cycloheptylcarbonyloxyethyl, 1-methylcyclopentylcarbonyloxymethyl, 1-methylcyclopentyloxycarbonyloxymethyl, 2-methyl-1-(1-methylcyclohexylcarbonyloxy)propyl, 1-(1-methylcyclohexylcarbonyloxy)propyl, 2-(1-methylcyclohexylcarbonyloxy)propyl, 1-(cyclohexylcarbonyloxy)propyl, 2-(cyclohexyl-carbonyloxy)propyl, 2-methyl-1-(1-methylcyclopentylcarbonyloxy)propyl, 1-(1-methylcyclopentylcarbonyloxy)propyl, 2-(1-methylcyclopentylcarbonyloxy)propyl, 1-(cyclopentylcarbonyloxy)propyl, 2-(cyclopentylcarbonyloxy)propyl, 1-(1-methylcyclopentylcarbonyloxy)ethyl, 1-(1-methylcyclopentylcarbonyloxy)propyl, adamantyloxycarbonyloxymethyl, adamantylcarbonyloxymethyl, 1-adamantyloxycarbonyloxyethyl and 1-adamantylcarbonyloxyethyl groups, preferably the 1-cyclohexylcarbonyloxyethyl or 1-cyclopentylcarbonyloxyethyl group;
cycloalkylalkoxycarbonyloxyalkyl groups in which the alkoxy group has a single cycloalkyl substituent, the cycloalkyl substituent having from 3 to 10, preferably from 3 to 7, carbon atoms and mono- or poly- cyclic, for example the cyclopropylmethoxycarbonyloxymethyl, cyclobutylmethoxycarbonyloxymethyl, cyclopentylmethoxycarbonyloxymethyl, cyclohexylmethoxycarbonyloxymethyl, 1-(cyclopropylmethoxycarbonyloxy)ethyl, 1-(cyclobutylmethoxycarbonyloxy)ethyl, 1-(cyclopentylmethoxycarbonyloxy)ethyl and 1-(cyclohexylmethoxycarbonyloxy)ethyl groups;
terpenylcarbonyloxyalkyl and terpenyloxycarbonyloxyalkyl groups, in which the terpenyl group is as exemplified above, and is preferably a cyclic terpenyl group, for example the 1-(menthyloxycarbonyloxy)ethyl, 1-(menthylcarbonyloxy)ethyl, menthyloxycarbonyloxymethyl, menthylcarbonyloxymethyl, 1-(3-pinanyloxycarbonyloxy)ethyl, 1-(3-pinanylcarbonyloxy)ethyl, 3-pinanyloxycarbonyloxymethyl and 3-pinanylcarbonyloxymethyl groups;
5-alkyl or 5-phenyl [which may be substituted by at least one of substituents C, defined and exemplified above] (2-oxo-1,3-dioxolen- 4-yl)alkyl groups in which each alkyl group (which may be the same or different) has from 1 to 6, preferably from 1 to 4, carbon atoms, for example the (5-methyl-2-oxo-1,3-dioxolen-4-yl)methyl, (5-phenyl-2-oxo-1,3-dioxolen-4-yl)methyl, (5-isopropyl-2-oxo-1,3-dioxolen-4-yl)methyl, (5-t-butyl-2-oxo-1,3-dioxolen-4-yl)methyl and 1-(5-methyl-2-oxo-1,3-dioxolen-4-yl)ethyl groups; and
other groups, especially groups which are easily removed in vivo such as the phthalidyl, indanyl and 2-oxo-4,5,6,7-tetrahydro-1,3-benzodioxoien-4-yl groups.

Substituents H are selected from hydroxy groups, carboxy groups, carbamoyl groups, cyano groups, halogen atoms, nitro groups, amino groups, alkyl groups having from 1 to 6 carbon atoms and alkoxy groups having from 1 to 6 carbon atoms.

When substituent H represents an alkyl group having from 1 to 6 carbon atoms, this may be as exemplified above in relation to the unsubstituted alkyl groups.

When substituent H represents an alkoxy group having from 1 to 6 carbon atoms, this may be any such group as exemplified in relation to substituents A, hereinabove.

Of the above groups, we especially prefer those groups which can be removed easily in vivo, and most preferably the aliphatic acyloxyalkyl groups (especially the pivaloyloxymethyl group), alkoxycarbonyloxyalkyl groups (especially the 1-isopropoxycarbonyloxyethyl group), cycloalkylcarbonyloxyalkyl groups (especially the 1-methylcyclohexylcarbonyloxymethyl and 1-cyclohexylcarbonyloxyethyl groups), phthalidyl groups and (5-substituted 2-oxo-1,3-dioxolen-4-yl)methyl groups [especially the (5-methyl-2-oxo-1,3-dioxolen-4-yl)methyl] group] .

In general, in the compounds of the present invention, R¹ may represent a hydrogen atom or a methyl group, preferably a methyl group.

The compounds of the present invention can form salts. Examples of such salts include: salts with an alkali metal, such as sodium, potassium or lithium; salts with an alkaline earth metal, such as barium or calcium; salts with another metal, such as magnesium or aluminium; ammonium salts; organic base salts, such as a salt with triethylamine, diisopropylamine, cyclohexylamine or dicyclohexylamine; and salts with a basic amino acid, such as lysine or arginine. Also, where the compound of the present invention contains a basic group in its molecule, it can form acid addition salts. Examples of such acid addition salts include: salts with mineral acids, especially hydrohalic acids (such as hydrofluoric acid, hydrobromic acid, hydroiodic acid or hydrochloric acid), nitric acid, carbonic acid, sulphuric acid or phosphoric acid; salts with lower alkylsulphonic acids, such as methanesulphonic acid, trifluoromethane- sulphonic acid or ethanesulphonic acid; salts with arylsulphonic acids, such as benzenesulphonic acid or p-toluenesulphonic acid; salts with organic carboxylic acids, such as acetic acid, fumaric acid, tartaric acid, oxalic acid, maleic acid, malic acid, succinic acid, benzoic acid, mandelic acid, ascorbic acid, lactic acid, gluconic acid or citric acid; and salts with amino acids, such as glutamic acid or aspartic acid.

The compounds of the present invention necessarily contain at least one and possibly several asymmetric carbon atoms in their molecules, and can thus form optical isomers. Although these are all represented herein by a single molecular formula, the present invention includes both the individual, isolated isomers and mixtures, including racemates thereof. Where stereospecific synthesis techniques are employed or optically active compounds are employed as starting materials, individual isomers may be prepared directly; on the other hand, if a mixture of isomers is prepared, the individual isomers may be obtained by conventional resolution techniques. The isomers preferably include compounds wherein R¹ represents a methyl group and the 1-position is of the R-configuration; compounds in which the 5- and 6-positions are of the (5S,6S) configuration, which is the same configuration as that of thienamycin; and the α-position having the hydroxy group of the 6-position substituent is of the R-configuration.

Of the compounds of the present invention, a preferred class of compounds are those compound of formula (I) and pharmaceutically acceptable salts and esters thereof, in which:
R¹ represents a hydrogen atom or a methyl group;
R² represents a hydrogen atom, an unsubstituted alkyl group having from 1 to 3 carbon atoms, a substituted alkyl group which has from 1 to 3 carbon atoms and which is substituted by at least one of substituents A¹, defined below, an alkenyl group having 3 or 4 carbon atoms, an alkynyl group having 3 or 4 carbon atoms, or a group of formula -C(=NH)R⁰,
   where R⁰ represents a hydrogen atom or an alkyl group having from 1 to 3 carbon atoms;
R³ represents a hydrogen atom or a negative ion; and
Q represents a group of formula (Q-VII) or (Q-VIII) defined above, wherein:
   R²⁰, R²¹ and R²² are independently selected from the group consisting of hydrogen atoms and unsubstituted alkyl groups having from 1 to 3 carbon atoms;
      or
   R²⁰ and R²¹ or R²⁰ and R²² together represent a group of formula -(CH₂)ₛ-(W)_{w}, -(CH₂)ₜ-, where s is 1 or 2, t is 1 or 2, W represents an oxygen or sulphur atom and w' is 0 or 1, provided that (s + w' + t) is 2, 3 or 4;
   R²³ and R²⁴ are independently selected from the group consisting of hydrogen atoms, halogen atoms, unsubstituted alkyl groups having from 1 to 3 carbon atoms, substituted alkyl groups which have from 1 to 3 carbon atoms and which are substituted by at least one of substituents C¹ defined below, hydroxy groups, carboxy groups, carbamoyl groups, amino groups, cyano groups and carbamoyloxy groups;
   n³ is 1, 2 or 3;
   R²⁵ represents a hydrogen atom, an unsubstituted alkyl group having from 1 to 3 carbon atoms, a substituted alkyl group which has from 1 to 3 carbon atoms and which is substituted by at least one of substituents C¹, defined below, an alkenyl group having 3 or 4 carbon atoms, or an alkynyl group having 3 or 4 carbon atoms;
   R²⁶ represents a hydrogen atom, a halogen atom, an unsubstituted alkyl group having from 1 to 3 carbon atoms, a substituted alkyl group which has from 1 to 3 carbon atoms and which is substituted by at least one of substituents C¹, defined below, a hydroxy group, a carboxy group, a carbamoyl group, an amino group, a cyano group or a carbamoyloxy group;
   n⁴ is 0, 1 or 2;
   p² is 0 or 1;
   substituents A¹ are selected from: hydroxy groups, carboxy groups, carbamoyl groups, carbamoyloxy groups, cyano groups, halogen atoms and amino groups; and
   substituents C¹ are selected from: hydroxy groups, carboxy groups, carbamoyl groups, halogen atoms and amino groups.

A more preferred class of compounds are those compounds of formula (I) and pharmaceutically acceptable salts and esters thereof, in which:
R¹ represents a hydrogen atom or a methyl group;
R² represents a hydrogen atom, an unsubstituted alkyl group having from 1 to 3 carbon atoms, a substituted alkyl group which has from 1 to 3 carbon atoms and which is substituted by at least one of substituents A², defined below, or a group of formula -C(=NH)R⁰,
   where R⁰ represents a hydrogen atom or an alkyl group having from 1 to 3 carbon atoms;
R³ represents a hydrogen atom or a negative ion; and
Q represents a group of formula (Q-VII) or (Q-VIII) defined above, wherein:
   R²⁰, R²¹ and R²² are independently selected from the group consisting of hydrogen atoms and unsubstituted alkyl groups having from 1 to 3 carbon atoms;
      or
   R²⁰ and R²¹ or R²⁰ and R²² together represent a group of formula - (CH₂)ₛ- (W)_{w,}- (CH₂)ₜ-_{,} where s is 1 or 2, t is 1 or 2, W represents an oxygen or sulphur atom and w' is 0 or 1, provided that (s + w' + t) is 2, 3 or 4;
   R²³ and R²⁴ are independently selected from the group consisting of hydrogen atoms, halogen atoms, hydroxy groups, carbamoyl groups, carboxy groups, unsubstituted alkyl groups having from 1 to 3 carbon atoms and substituted alkyl groups which have from 1 to 3 carbon atoms and which are substituted by at least one of hydroxy groups, amino groups and carbamoyl groups;
   n³ is 1, 2 or 3;
   R²⁵ represents a hydrogen atom or a methyl group;
   R²⁶ represents a hydrogen atom;
   n⁴ is 0, 1 or 2;
   p² is 0 or 1; and
   substituents A² are selected from: hydroxy groups, carboxy groups, carbamoyl groups, carbamoyloxy groups, halogen atoms and amino groups.

Of these, we still more prefer those in which Q represents a group of formula (Q-VII).

Of all of these compounds, the most preferred are those in which R¹ represents a methyl group.

Specific examples of the compounds of the present invention are shown in the following formula (I-2), is as defined in Tables 7 and 8.

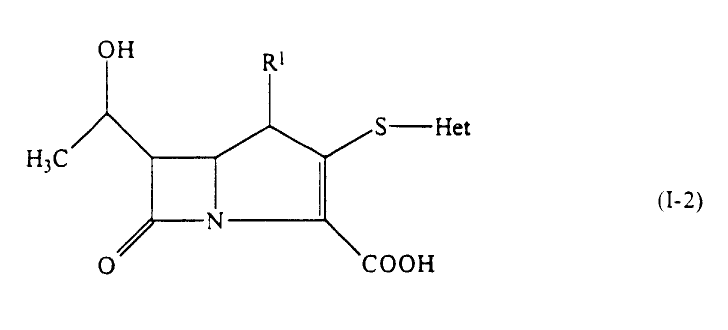

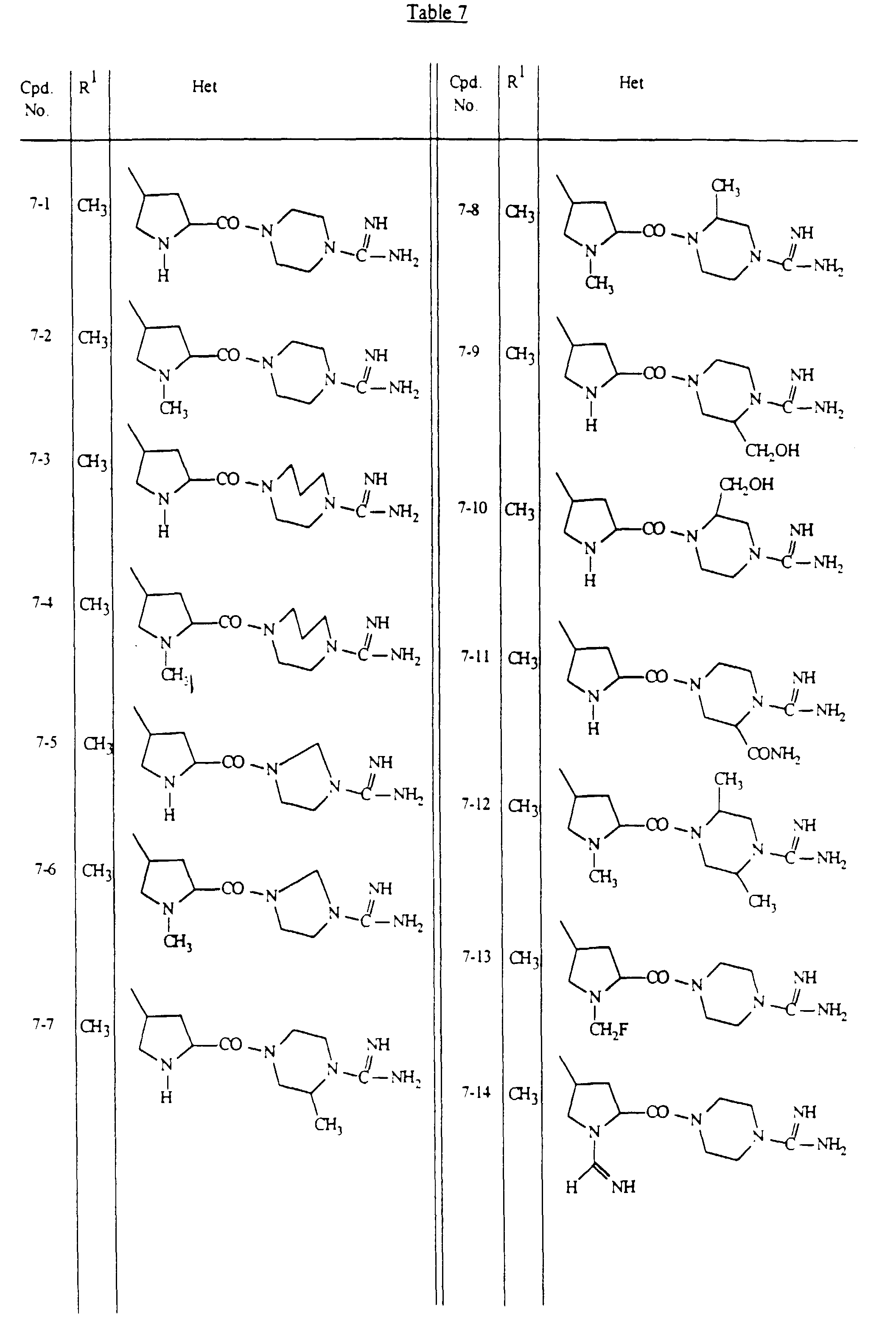

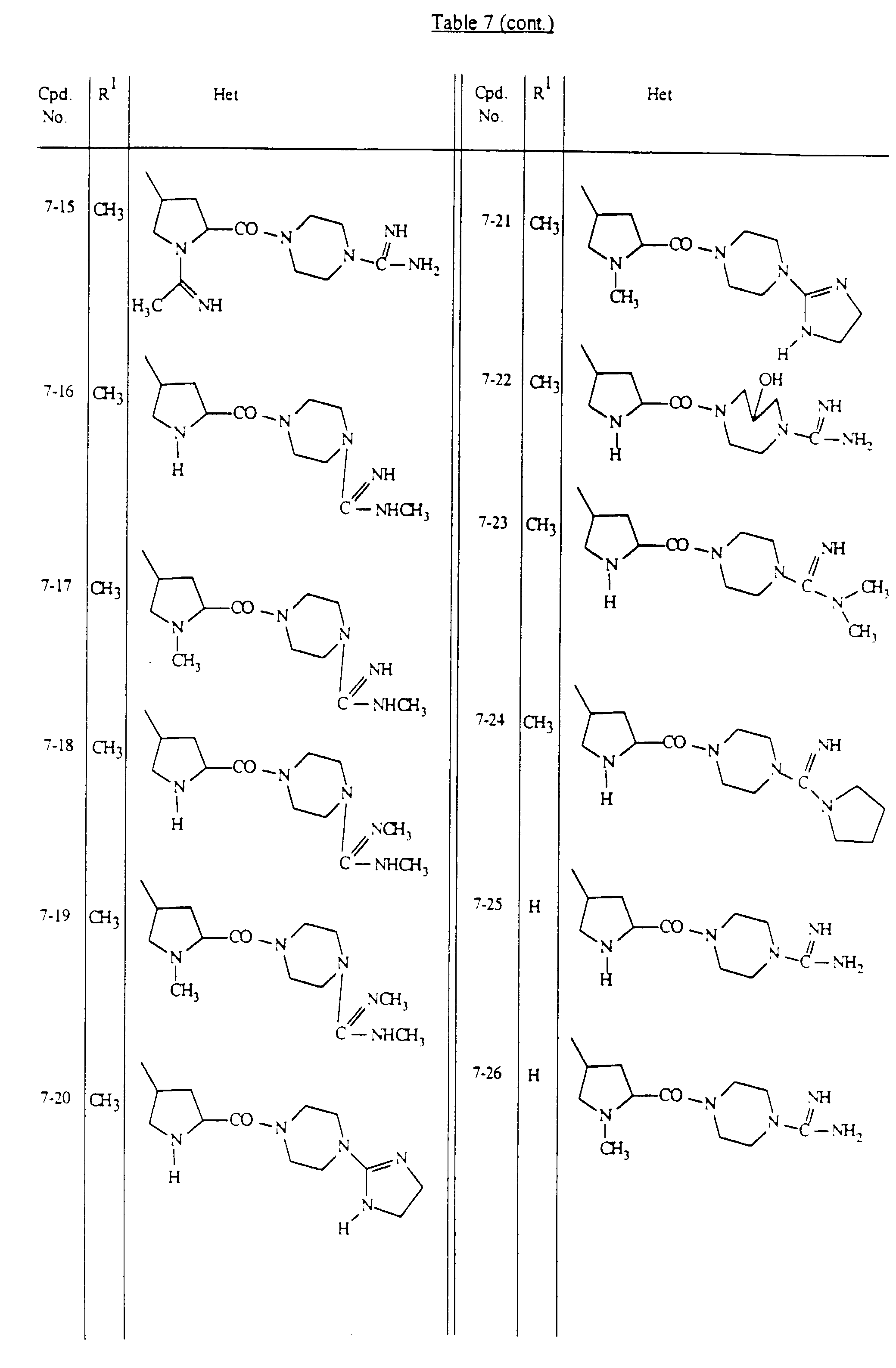

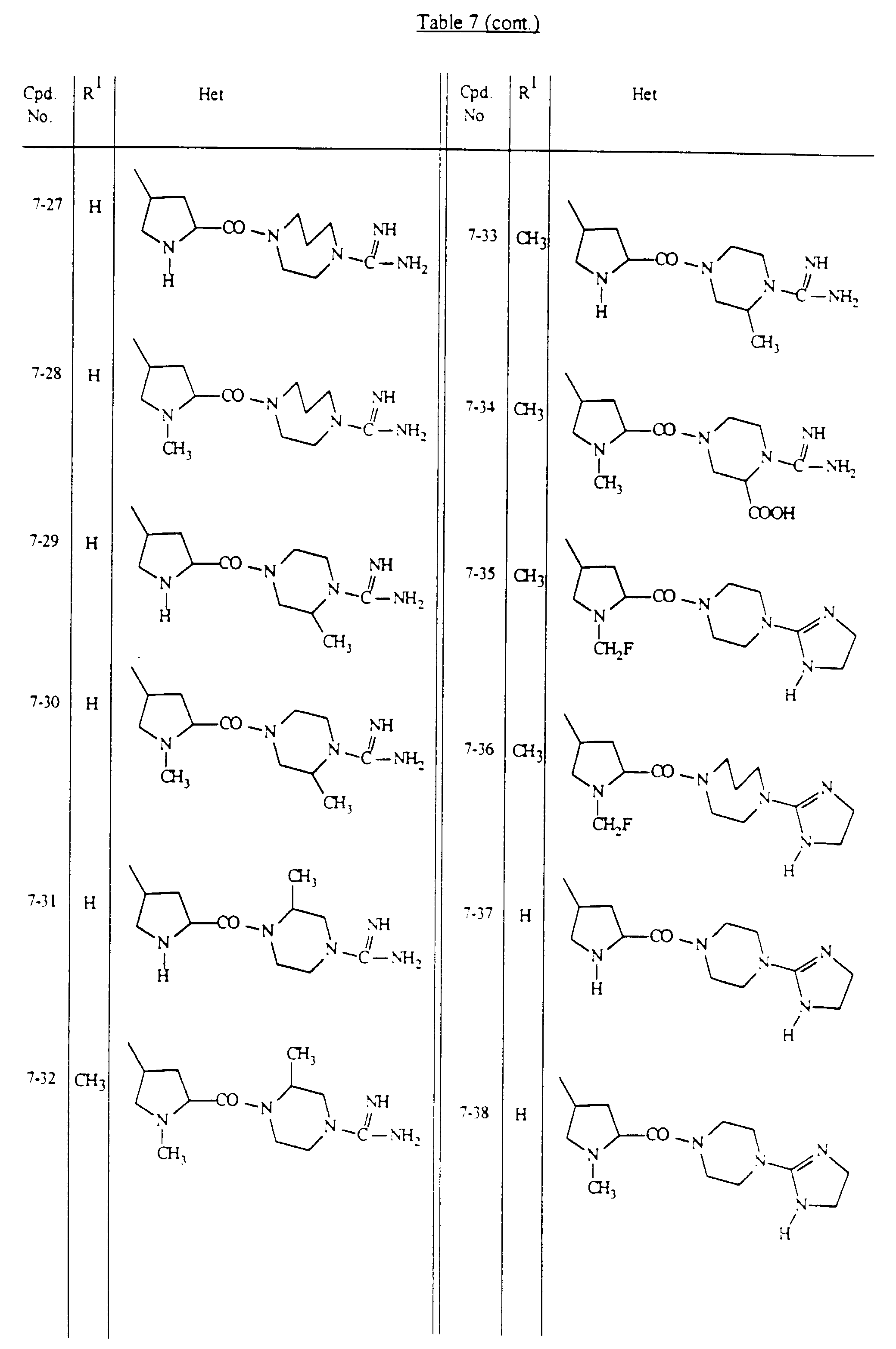

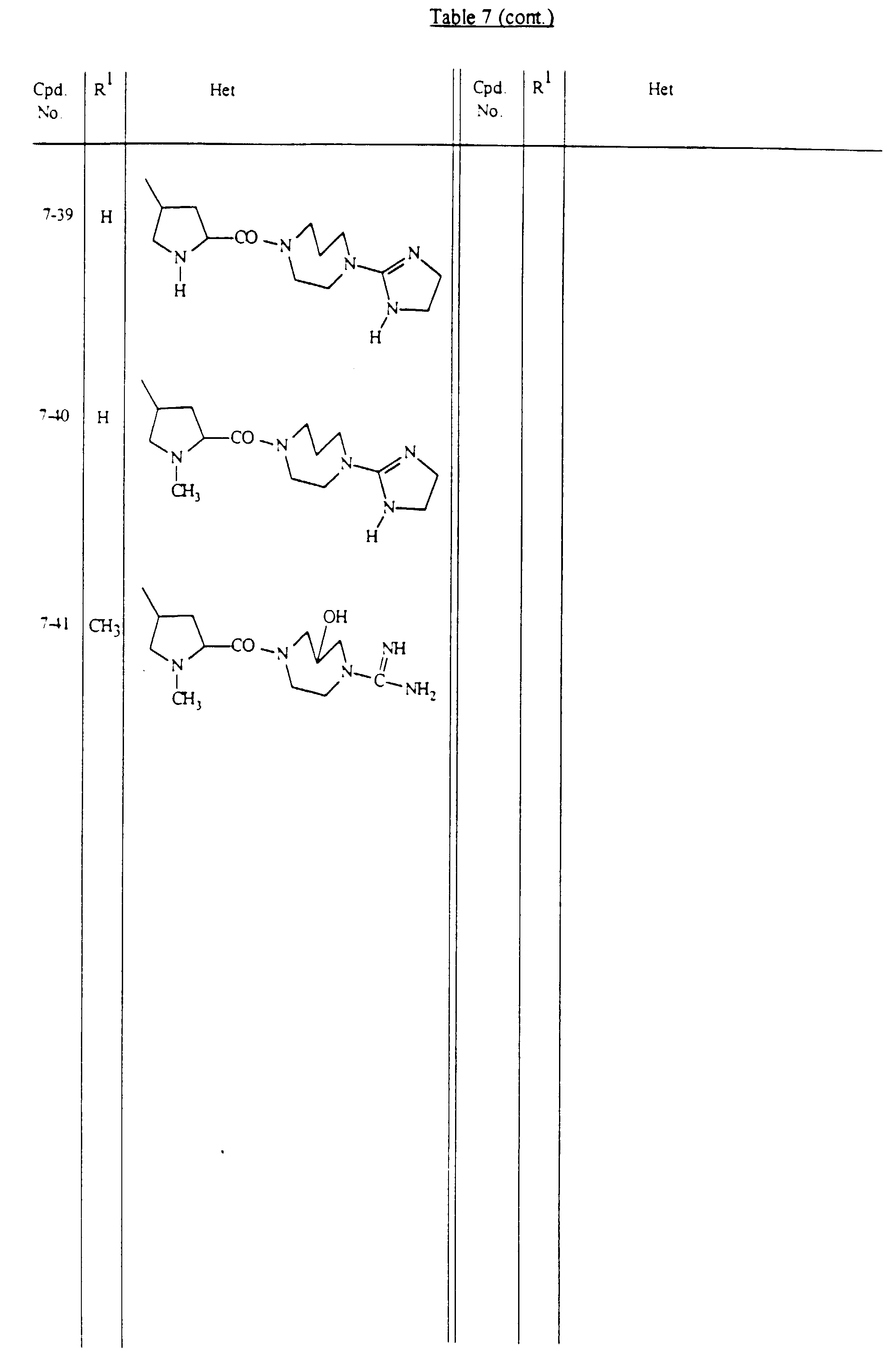

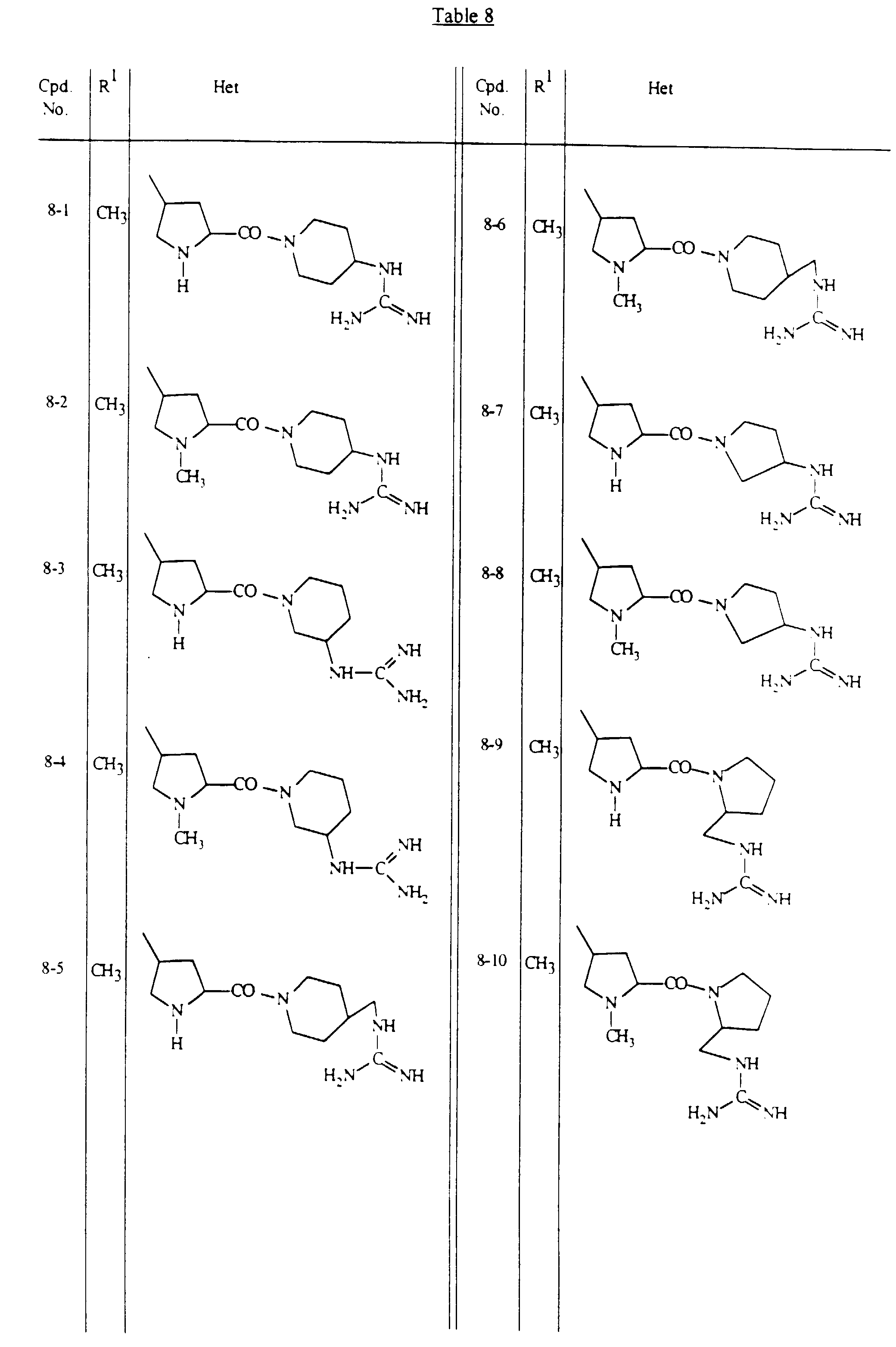

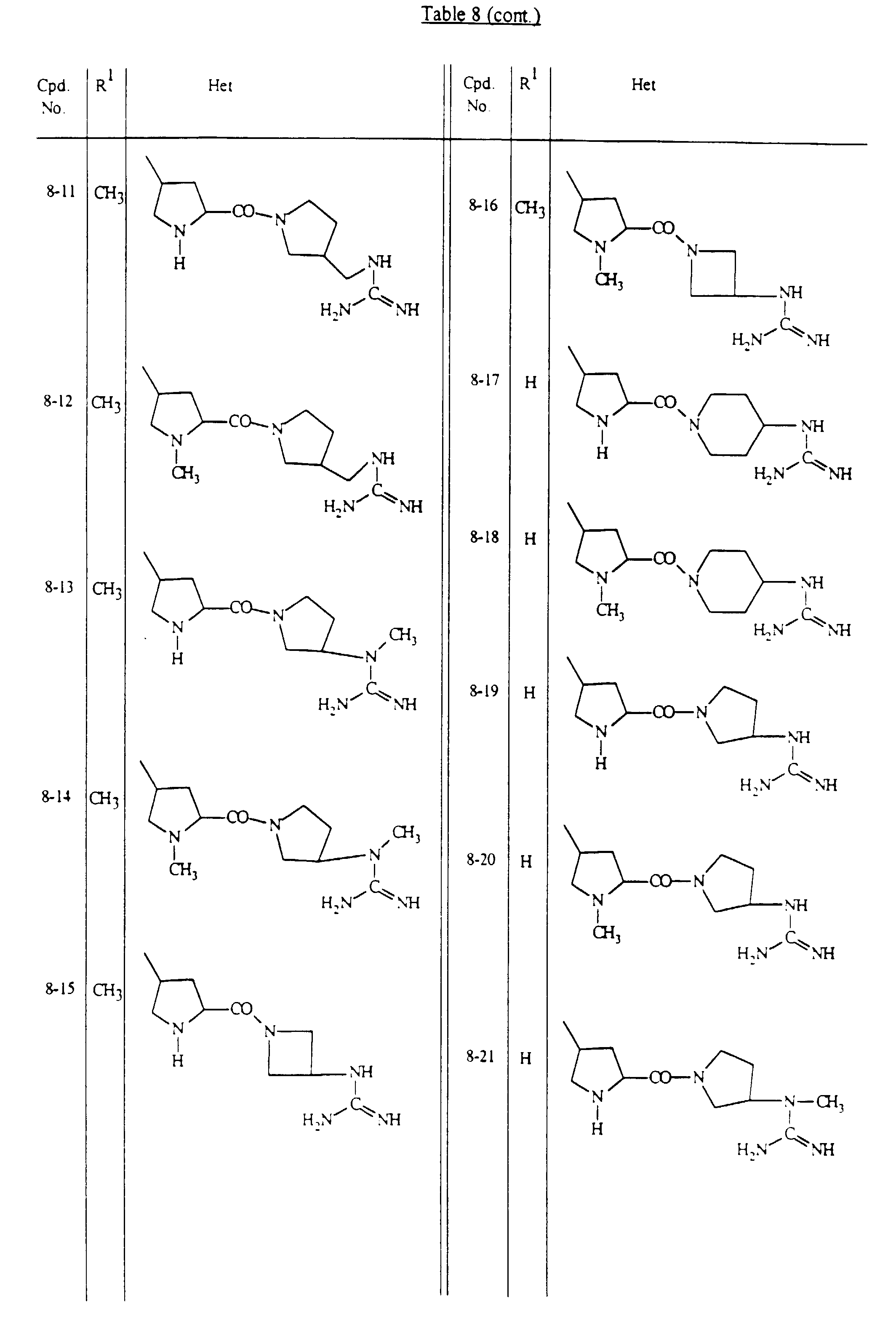

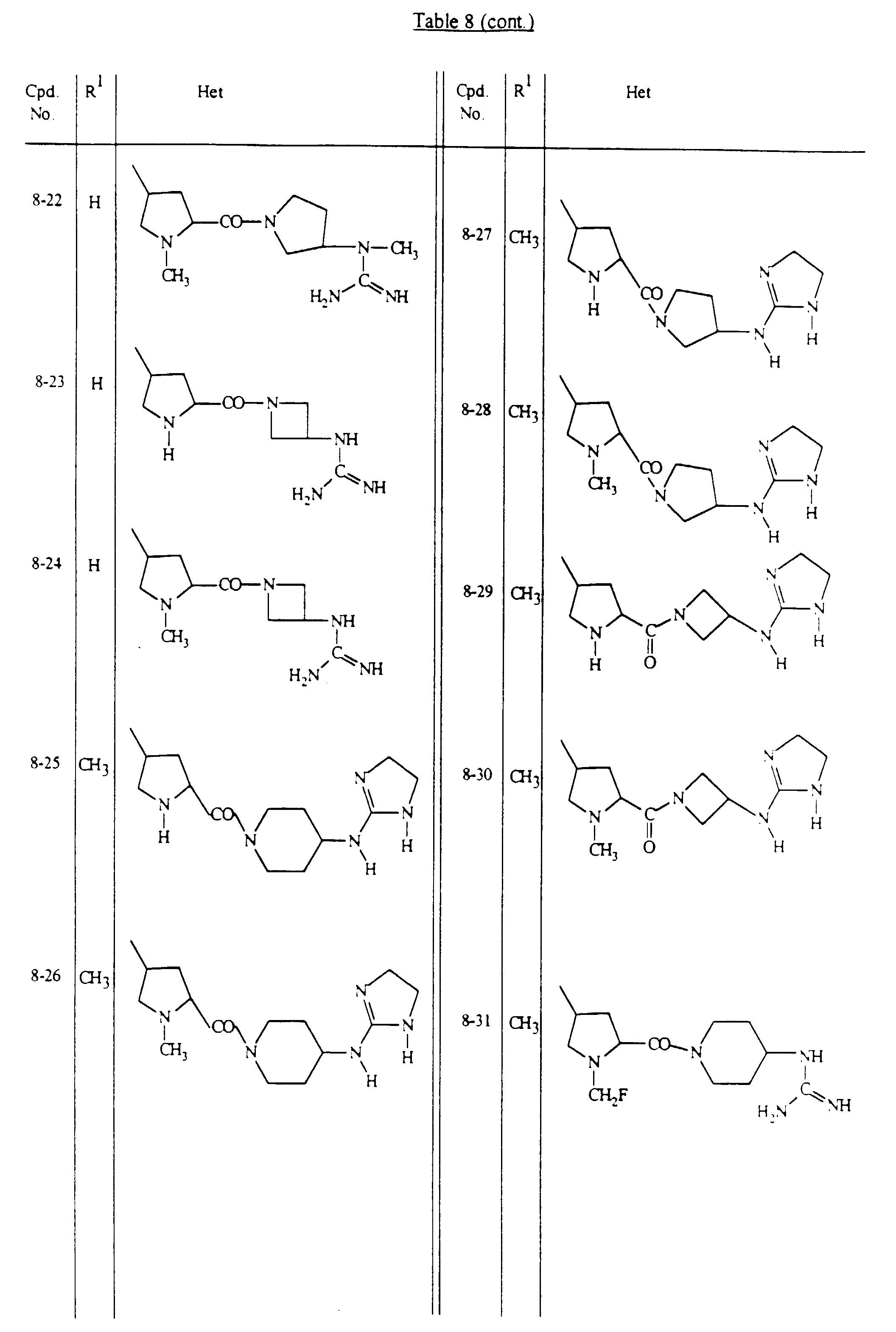

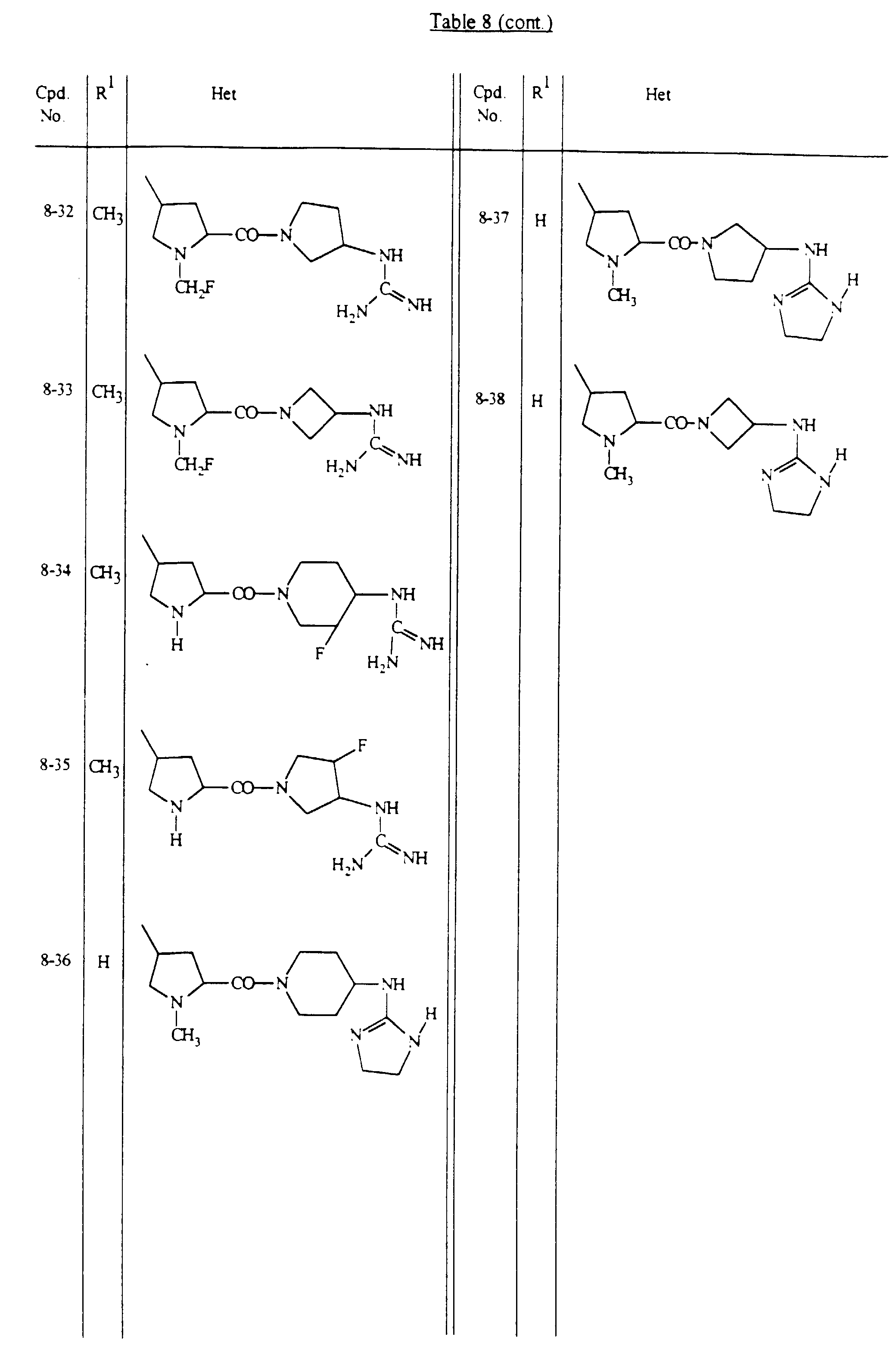

Of the specific compounds of the present invention, preferred compounds are:
2-[2-(4-Amidinopiperazin-1-ylcarbonyl)pyrrolidin-4-ylthio]-6-(1-hydroxyethyl)-1-methyl-1-carbapen-2-em-3-carboxylic acid;
2-[2-(4-Amidinopiperazin-1-ylcarbonyl)-1-methylpyrrolidin-4-ylthio]-6-(1-hydroxyethyl)-1-methyl-1-carbapen-2-em-3-carboxylic acid;
2-[2-(4-Amidinohomopiperazin-1-ylcarbonyl)pyrrolidin-4-ylthio]-6-(1-hydroxyethyl)-1-methyl-1-carbapen-2-em-3-carboxylic acid; and
2-[2-(4-Amidinohomopiperazin-1-ylcarbonyl)-1-methyl-pyrrolidin-4-ylthio]-6-(1-hydroxyethyl)-1-methyl-1-carbapen-2-em-3-carboxylic acid;
and pharmaceutically acceptable salts thereof.

The compounds of the present invention may be prepared by a variety of processes well known in the art for the preparation of compounds of this type. For example, in general terms, they may be prepared by reacting a carbapenem compound of formula (II) : 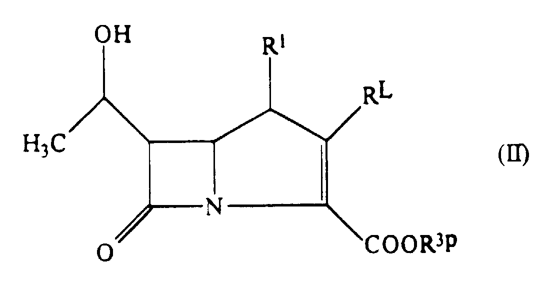 (in which R¹ is as defined above, R^{L} represents a sulphonyloxy or phosphoryloxy group or a group of formula -S(O)R^{L1}, R^{L1} represents an alkyl group, a haloalkyl group, an alkanoylaminoalkyl group, an alkenoylamino- alkyl group, an aryl group or an aromatic heterocyclic group, and R^{3p} represents a carboxy-protecting group) with a pyrrolidine derivative of formula (III): 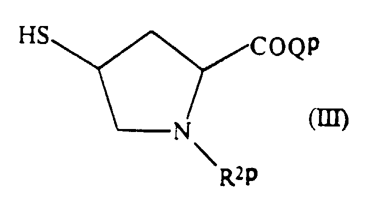 (in which R^{2p} represents any of the groups or atoms represented by R² or any such group or atom which has been protected, and Q^{p} represents any of the groups or atoms represented by Q or any such group or atom which has been protected and, where the group Q^{p} contains a quaternary nitrogen atom, the compound is accompanied by a balancing anion),
and, if necessary, removing any protecting groups,
and optionally salifying and/or esterifying the product.

In more detail, the reactions involved may be as illustrated in the following Reaction Schemes A and B:

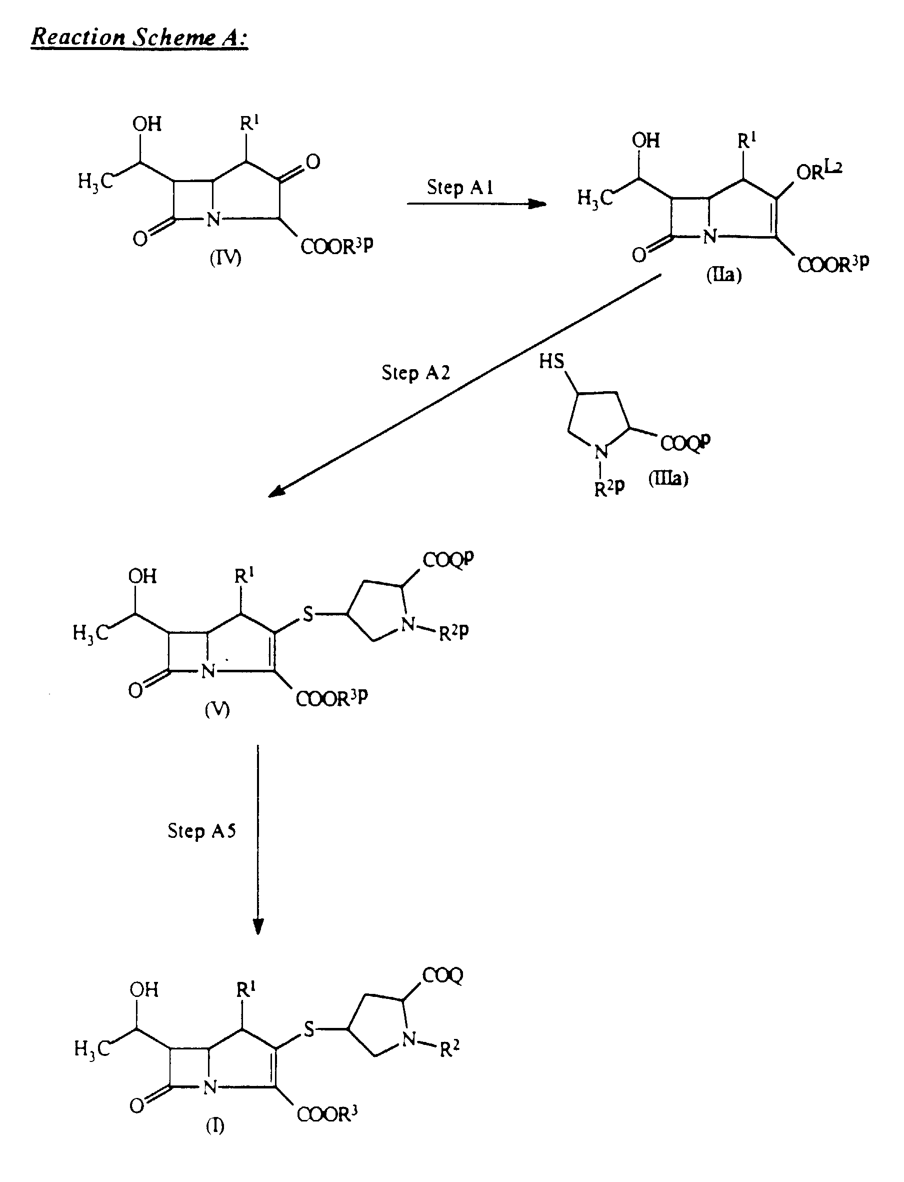

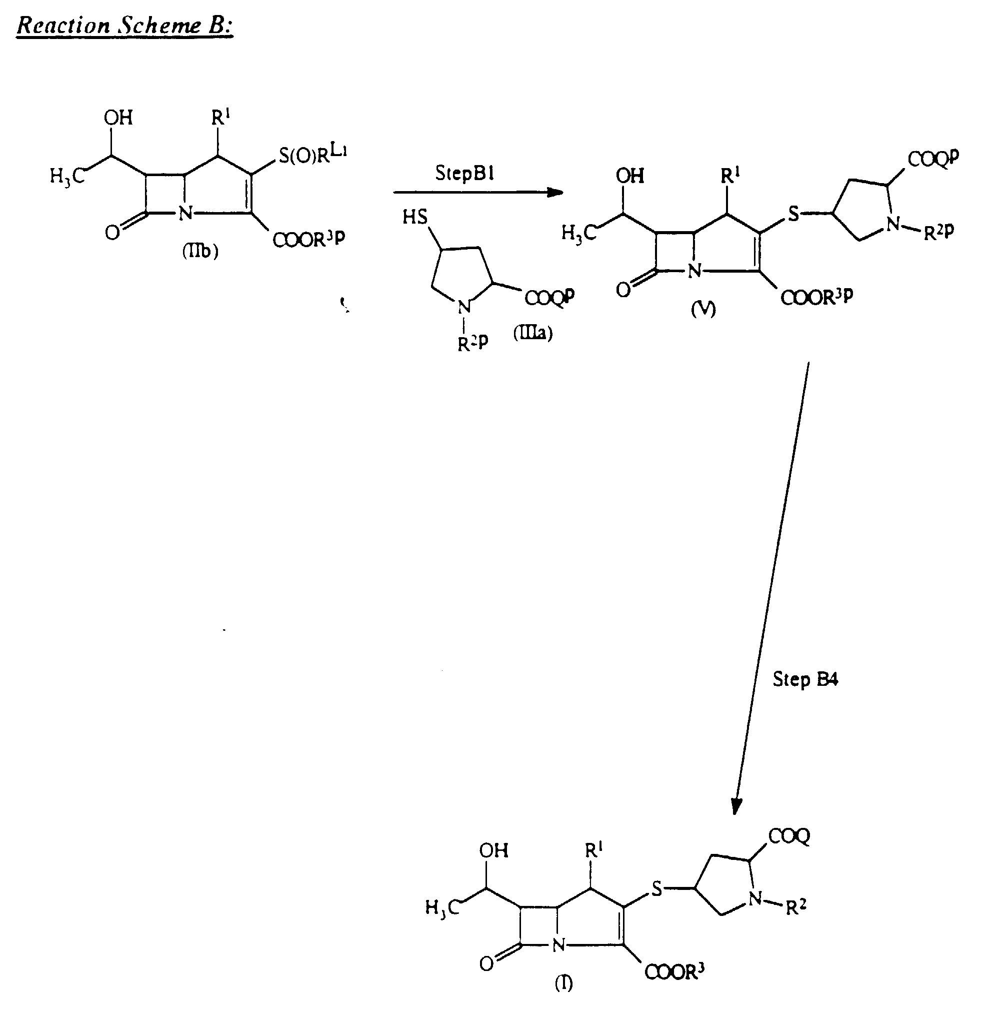

In the above formulae, R¹, R², R³, R^{2p}, R^{3p}, Q^{p} and Q are as defined above.

R^{L1} represents:
an alkyl group, such as a methyl, ethyl, propyl or isopropyl group;
a haloalkyl group, such as a fluoromethyl, chloromethyl, fluoroethyl, chloroethyl, fluoropropyl, difluoromethyl, difluoroethyl, dichloroethyl, trifluoromethyl or trifluoroethyl group;
a 2-acetamidoethyl group;
a 2-acetamidovinyl group;
an optionally substituted aryl group, such as a phenyl or naphthyl group which may be unsubstituted or may have from 1 to 3 substituents which may be the same or different from one another, for example: fluorine, chlorine, bromine, methyl, ethyl, propyl, isopropyl, methoxy, ethoxy, propoxy, isopropoxy, methoxycarbonyl, ethoxycarbonyl, t-butoxycarbonyl, nitro, carbamoyl, mono- and di-substituted alkylcarbamoyl (where the alkyl group is, for example, methyl, ethyl or propyl), hydroxy or cyano;
or an optionally substituted aromatic heterocyclic group, such as a pyridyl and pyrimidyl group, which may be unsubstituted or may have from 1 to 3 substituents which may be the same or different from one another, for example: fluorine, chlorine, bromine, methyl, ethyl, propyl and isopropyl.

R^{L2} represents:
an alkanesulphonyl group, such as a methanesulphonyl, trifluoromethanesulphonyl, ethanesulphonyl, propane- sulphonyl, isopropanesulphonyl or butanesulphonyl group;
an arylsulphonyl group, such as a phenylsulphonyl, tolylsulphonyl, e.g. p-tolylsulphonyl, or 1- or 2-naphthylsulphonyl group;
a dialkylphosphoryl group, such as a dimethylphosphoryl, diethylphophoryl, dipropylphosphoryl, diisopropylphosphoryl, dibutylphosphoryl or dipentylphosphoryl group;
or a diarylphosphoryl group, such as a diphenylphosphoryl or ditolylphosphoryl group.

If Q^{p} or R^{3p} includes a protecting group, this may be selected from the many such groups well known to those skilled in the art and commonly used for the protection of hydroxy groups, imino groups, amino groups or carboxy groups. Such groups are described fully in many standard texts, for example T.W. Greene, "Protective Groups in Organic Synthesis", published by John Wiley & Son. Examples of such groups typically include the p-nitrobenzyloxycarbonyl and p-nitrobenzyl groups.

As defined above, R^{3p} represents a protecting group for the carboxy group, such as an alkyl group, e.g. a methyl, ethyl or t-butyl group; an aralkyl group, e.g. a benzyl, diphenylmethyl, 4-nitrobenzyl or 2-nitrobenzyl group; an alkenyl group, e.g. an allyl, 2-chloroallyl or 2-methylallyl group; a haloalkyl group, e.g. a 2,2,2-trichloroethyl, 2,2-dibromoethyl or 2,2,2-tribromoethyl group; or a 2-trimethylsilylethyl group.

In Step A1 of Reaction Scheme A, a carbapenam compound of formula (IV) is converted to a carbapenem compound of formula (IIa) by reaction with an active sulphonyl or phosphoryl compound containing a group corresponding to the group R^{L2}, for example an alkanesulphonic anhydride, an arylsulphonic anhydride, a dialkylphosphoryl halide or a diarylphosphoryl halide.

This reaction is normally and preferably effected in the presence of a base. There is no particular restriction on the nature of the base employed, provided that it has no adverse effect on other parts of the molecule, notably the β-lactam ring, and preferred examples include organic bases such as triethylamine, diisopropylethylamine and 4-dimethylaminopyridine.

Examples of sulphonyl or phosphoryl compounds which may be employed in this reaction include: alkanesulphonic anhydrides, such as methanesulphonic anhydride, trifluoromethanesulphonic anhydride and ethanesulphonic anhydride; arylsulphonic anhydrides, such as benzene- sulphonic anhydride and p-toluenesulphonic anhydride; dialkylphosphoryl halides, such as dimethylphosphoryl chloride and diethylphosphoryl chloride; and diaryl- phosphoryl halides, such as diphenylphosphoryl chloride and diphenylphosphoryl bromide. Of these reagents, we particularly prefer p-toluenesulphonic anhydride and diphenylphosphoryl chloride.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: halogenated hydrocarbons, such as methylene chloride, 1,2-dichloroethane and chloroform; nitriles, such as acetonitrile; and amides, such as dimethylformamide and dimethylacetamide.

The reaction can take place over a wide range of temperatures, and the precise reaction temperature is not critical to the invention. In general, we find it convenient to carry out the reaction at a relatively low temperature, for example from -20°C to 40°C, in order to suppress any side reactions. The time required for the reaction may also vary widely, depending on many factors, notably the reaction temperature and the nature of the reagents and solvent employed. However, provided that the reaction is effected under the preferred conditions outlined above, a period of from 10 minutes to 5 hours will usually suffice.

The compound of formula (IIa) thus obtained is not normally isolated; instead, in Step A2, the reaction mixture is reacted, without any isolation, with a mercaptan of formula (IIIa) in the presence of a base to obtain a compound of formula (V). The resulting compound may then, if necessary, be subjected, in Step A5, to a deprotection reaction to eliminate any protecting group in the groups represented by R^{3p} or Q^{p} and thus to prepare the desired compound of formula (I).

There is no particular limitation on the nature of the base which may be employed in Step A2, provided that it has no adverse effect on other parts of the molecule, notably the β-lactam ring, and preferred examples include: organic bases, such as triethylamine and diisopropylethylamine; and inorganic bases, such as potassium carbonate and sodium carbonate. The reaction can take place over a wide range of temperatures, and the precise reaction temperature is not critical to the invention. In general, we find it convenient to carry out the reaction at a temperature of from -20°C to room temperature. The time required for the reaction may also vary widely, depending on many factors, notably the reaction temperature and the nature of the reagents and solvent employed. However, provided that the reaction is effected under the preferred conditions outlined above, a period of from 30 minutes to 5 days will usually suffice.

After completion of the reaction, the desired compound of formula (V) can be collected from the reaction mixture by any conventional procedure. For example, in one suitable recovery procedure, the solvent is removed from the reaction mixture by distillation to obtain the desired compound. Alternatively, in the case of the non-quaternary compounds, the solvent is removed, preferably by evaporation, the residue is extracted with an organic solvent, and the extract is washed with water and dried, after which the solvent is removed by evaporation. The compound thus obtained can be further purified, if necessary, by conventional procedures, such as recrystallisation, reprecipitation or the various chromatography techniques, notably column chromatography. If desired, the compound can be purified by subjecting the reaction mixture directly to reprecipitation. Alternatively, if desired, the compound of formula (V) can be subjected to the subsequent carboxy deprotection reaction in Step A5 without isolation.

If necessary, in Step A5, the compound of formula (V) obtained in Step A2 can be converted to a carboxylic acid derivative of formula (I) by eliminating the protecting group R^{3p} for the carboxy group by conventional means. The nature of the reaction employed for the elimination of the protecting group will, of course, vary, depending upon the nature of the protecting group, as is well known in the art. For example, where the protecting group can be eliminated by reduction (as can the haloalkyl groups, the aralkyl groups, and the benzyhydryl group), this can be achieved by bringing the compound of formula (V) into contact with a reducing agent. Examples of preferred reducing agents which may be employed in this reaction include: zinc and acetic acid when the carboxy-protecting group is, for example, a haloalkyl group (such as a 2,2-dibromoethyl or 2,2,2,-trichloroethyl group); or, when the protecting group is, for example, an aralkyl group (such as a benzyl or 4-nitrobenzyl group) or a benzhydryl group, it is possible to use as the reducing agent either an alkali metal sulphide (such as sodium sulphide or potassium sulphide) or hydrogen in the presence of a reducing catalyst (such as palladium-on-carbon). The reducing reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: alcohols, such as methanol or ethanol; ethers, such as tetrahydrofuran or dioxan; fatty acids, such as acetic acid; and mixtures of any one or more of such organic solvents with water. The reaction can take place over a wide range of temperatures, and the precise reaction temperature is not critical to the invention. In general, we find it convenient to carry out the reaction at a temperature of from 0°C to approximately room temperature. The time required for the reaction may also vary widely, depending on many factors, notably the reaction temperature and the nature of the reagents and solvent employed. However, provided that the reaction is effected under the preferred conditions outlined above, a period of from 5 minutes to 12 hours will usually suffice.

If Q^{p} and/or R^{2p} includes a protecting group for a hydroxy group, an imino group, an amino group or a carboxy group (for example a p-nitrobenzyloxycarbonyl group or a p-nitrobenzyl group), such a protecting group can be eliminated simultaneously with the elimination of the above-mentioned carboxy-protecting group (in the case where R^{3p} is a p-nitrobenzyl group).

After completion of the reaction, the desired compound of the present invention can be collected from the reaction mixture by conventional means. For example, one suitable recovery procedure comprises: removing insolubles precipitated from the reaction mixture by filtration; and then removing the solvent by distillation. The compound thus obtained can be further purified, if necessary, by conventional procedures, such as recrystallisation, reprecipitation or the various chromatography techniques, notably column chromatography.

The carboxy group of the compound thus obtained can be converted to an ester group, particularly to such a group which undergoes hydrolysis under physiological conditions, by conventional means. When R³ is an ester which undergoes hydrolysis under physiological conditions [such as an alkanoyloxyalkyl group, e.g. a pivaloyloxymethyl or acetoxymethyl group, an alkoxycarbonyloxyalkyl group, e.g. a 1-(ethoxycarbonyloxy)ethyl or 1-(isopropoxycarbonyloxy)ethyl group, or a phthalidyl, indanyl, methoxymethyl or 2-oxo-5-methyl-1,3-dioxolen-4-ylmethyl group], the compound of formula (I) need not be deprotected and can be administered directly to a patient, since such a compound can be hydrolyzed in a living body under physiological conditions.

Alternatively, the compound (V) can be prepared, as shown in Reaction Scheme B, from a compound (IIb). This compound of formula (IIb) can be synthesized by the method disclosed in Japanese Unexamined Patent Publication No. Sho-62-30781 (Kokai). The reaction of the compound of formula (IIb) with a mercaptan of formula (IIIa) in the presence of a base to give a compound of formula (V) is normally and preferably carried out in an inert solvent. There is no particular restriction on the nature of the solvent to be employed, provided that it has no adverse effect on the reaction or on the reagents involved and that it can dissolve the reagents, at least to some extent. Examples of suitable solvents include: tetrahydrofuran, acetonitrile, dimethylformamide, dimethyl sulphoxide, water and mixtures of any two or more of these solvents. There is also no particular limitation on the nature of the base employed, provided that it does not affect other portions of the compound, particularly the β-lactam ring, and examples include: organic bases, such as diisopropylethylamine, triethylamine, N-methylpiperidine or 4-dimethylaminopyridine; and inorganic bases, such as potassium carbonate or sodium hydrogencarbonate. The reaction can take place over a wide range of temperatures, and the precise reaction temperature is not critical to the invention. In general, we find it convenient to carry out the reaction at a relatively low temperature in order to suppress any side reaction, usually at a temperature of from -20°C to 40°C. The time required for the reaction may also vary widely, depending on many factors, notably the reaction temperature and the nature of the reagents and solvent employed. However, provided that the reaction is effected under the preferred conditions outlined above, a period of from 5 minutes to 5 days will usually suffice.

After completion of the reaction, the desired compound of formula (V) can be collected from the reaction mixture by conventional means, as described in relation to Reaction Scheme A. It may then be subjected to Step B4, which correspond to Step A5 of Reaction Scheme A.

Any of the compounds prepared as described above may be salified and/or esterified by conventional means well known in the art.

The compounds of the present invention exhibit excellent antibacterial activity with a broad antibacterial spectrum, and have the ability to inhibit the activity of β-lactamase, unlike most thienamycin-type compounds, which are liable to be metabolised in the mammalian body. The derivatives of the present invention, in addition, exhibit excellent stability against dehydropeptidase I, which is also known to catalyse the inactivation of compounds of the thienamycin type. The derivatives of the present invention showed a strong antibacterial activity against a wide range of pathogenic bacteria including Gram-positive bacteria such as Staphylococcus aureus, and Enterococcus faecalis, Gram-negative bacteria such as Escherichia coli, Shigella species, Streptococcus pneumoniae, Proteus species, Serratia species, Enterobacter species and Pseudomonas species, and anaerobic bacteria such as Bacteroides fragilis.

The antibacterial activity was determined by the agar plate dilution method, and the minimal inhibitory concentrations of a compound of the present invention against a variety of common pathogenic bacteria were determined and are shown in the following Table 14. In the Table, the compound of the present invention is identified by reference to the one of the following Examples which illustrates its preparation.

The microorganisms used are identified as follows:
A:Staphylococcus aureus 209P;
B:Escherichia coli NIHJ;
C:Pseudomonas aeruginosa 1001.

**Table 14**

| Compound of Example No. | Microorganism | | |
|---|---|---|---|
| | A | B | C |
| 12 | ≤0.01 | ≤0.01 | 0.1 |
| imipenem | ≤0.01 | 0.05 | 3.1 |

These results demonstrate that the compounds of the present invention have activities which are, in general, better than that of imipenem: moreover, they are, unlike imipenem, resistant to dehydropeptidase I and β-lactamase.

The carbapenem-3-carboxylic acid derivatives of the present invention, therefore, are useful as therapeutic agents for the treatment and prophylaxis of infections caused by these pathogenic bacteria. The compounds may be administered in any conventional form for this purpose, and the exact formulation used will depend on the disease to be treated, the age and condition of the patient and other factors, which are well known in the art. For example, for oral administration, the compounds may be formulated as tablets, capsules, granules, powders or syrups; and for parenteral administration, they may be formulated for intravenous injection or intramuscular injection. The dosage will vary widely, depending upon the age, body weight, symptoms and condition of the patient, as well as the mode of administration and times and routine of administration; however, for an adult human patient, a daily dosage of from about 100 mg to 3000 mg is recommended, and this may be administered as a single dose or in divided doses.

The preparation of certain of the compounds of the invention is further illustrated by the following Examples, and the preparation of certain starting materials is illustrated by the subsequent Preparations. All mesh sizes used herein are Tyler standard mesh.

### EXAMPLE 12

### (1R,5S,6S)-2-[(2S,4S)-2-(4-Amidinopiperazin-1-yl-carbonyl)pyrrolidin-4-ylthio]-6-[(1R)-1-hydroxyethyl]-1-methyl-1-carbapen-2-em-3-carboxylic acid

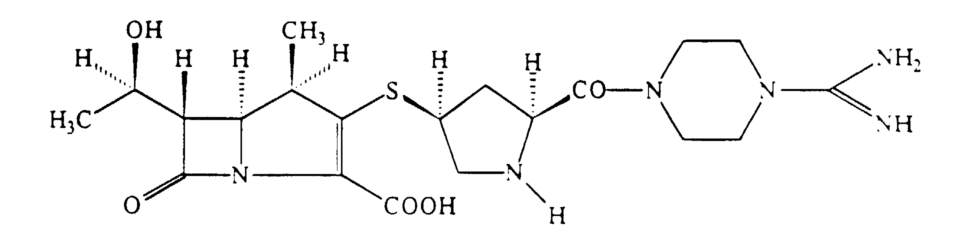

### 12(1) 4-Nitrobenzyl (1R,5S,6S)-6-[(1R)-1-hydroxyethyl]-1-methyl-2-[(2S,4S)-2-[4-(4-nitrobenzyloxycarbonyl-amidino)piperazin-1-ylcarbonyl]-1-(4-nitrobenzyloxycarbonyl)pyrrolidin-4-ylthio]-1-carbapen-2-em-3-carboxylate

290 µℓ of diphenylphosphoric acid chloride and 244 µℓ of diisopropylethylamine were added dropwise, whilst ice-cooling, to a solution of 471 mg of 4-nitrobenzyl (1R,5S,6S)-6-[(1R)-1-hydroxyethyl]-1-methyl-2-oxo-1-carbapenam-3-carboxylate in 5 ml of dry acetonitrile, and the resulting mixture was stirred at the same temperature for 1 hour. At the end of this time, a solution of 910 mg of (2S,4S)-4-mercapto-2-[4-(4-nitrobenzyloxycarbonylamidino)piperazin-1-ylcarbonyl]-1-(4-nitrobenzyloxycarbonyl)pyrrolidine trifluoromethanesulphonate (prepared as described in Preparation 1) in 7 ml of dry acetonitrile and 500 µℓ of diisopropylethylamine were added dropwise, whilst ice-cooling, to the mixture, and the resulting mixture was left to stand overnight at the same temperature. At the end of this time, the reaction mixture was concentrated by evaporation under reduced pressure, and the resulting residue was diluted with ethyl acetate, after which the mixture was washed with an aqueous solution of sodium hydrogencarbonate, with water and with an aqueous solution of sodium chloride. The ethyl acetate layer was dehydrated over anhydrous sodium sulphate and concentrated by evaporation under reduced pressure. The resulting residue was subjected to silica gel column chromatography [silica gel 60 (Art. 9385), manufactured by Merck, 150 ml], eluted with mixtures of ethyl acetate and acetonitrile in proportions of 8 : 2, 7 : 3 and 6 : 4, in that order. The fractions containing the desired compound were collected and concentrated by evaporation under reduced pressure, to obtain 691 mg of the title compound, as an amorphous powder.
Infrared Absorption Spectrum (KBr), νₘₐₓ cm⁻¹:
   1773, 1710, 1652, 1607, 1552, 1441, 1347.
Nuclear Magnetic Resonance Spectrum (hexadeuterated dimethyl sulphoxide + D₂O, 270 MHz) δ ppm:
   1.12 - 1.21 (6H, multiplet);
   1.62 - 1.78 (1H, multiplet);
   2.77 - 2.93 (1H, multiplet) ;
   3.11 - 4.30 (15H, multiplet);
   4.79 & 4.88 (together 1H, two triplets, J = 7.8 Hz);
   5.07 - 5.49 (6H, multiplet);
   7.52 - 7.73 (6H, multiplet);
   8.19 - 8.25 (6H, multiplet).

### 12(2) (1R,5S,6S)-2-[(2S,4S)-2-(4-Amidinopiperazin-1-yl-carbonyl)pyrrolidin-4-ylthio]-6-[(1R)-1-hydroxyethyl]-1-methyl-1-carbapen-2-em-3-carboxylic acid

680 mg of 4-nitrobenzyl (1R,5S,6S)-6-[(1R)-1-hydroxyethyl]-1-methyl-2-[(2S,4S)-2-[4-(4-nitrobenzyloxycarbonylamidino)piperazin-1-ylcarbonyl]-1-(4-nitrobenzyloxycarbonyl)pyrrolidin-4-ylthio]-1-carbapen-2-em-3-carboxylate [prepared as described in step (1) above] were dissolved in 40 ml of a 1 : 1 by volume mixture of tetrahydrofuran and water, and 950 mg of a 10% w/w palladium-on-carbon catalyst were added to the resulting solution. The mixture was then hydrogenated in an atmosphere of hydrogen at 28°C for 1 hour. At the end of this time, the catalyst was removed by filtration, and the filtrate was washed with diethyl ether. The aqueous layer was then concentrated by evaporation under reduced pressure, to 10 ml. The resulting solution was subjected to reverse phase silica gel column chromatography (Cosmosil 75C₁₈-prep, manufactured by Nacalai Tesque, 30 ml), eluted with mixtures of acetonitrile and water in proportions of 0 : 100, 2 : 98, 4 : 96 and 6 : 94, in that order. The fractions containing the desired compound were collected, concentrated by evaporation under reduced pressure, and freeze-dried to obtain 211 mg of the title compound as a powder.
Ultraviolet Absorption Spectrum (H₂O), λₘₐₓ nm:
   299.
Infrared Absorption Spectrum (KBr), νₘₐₓ cm⁻¹:
   1754, 1649, 1605, 1450, 1389, 1251.
Nuclear Magnetic Resonance Spectrum (270MHz, D₂O, internal standard: tetradeuterated sodium trimethylsilylpropionate) δ ppm:
   1.22 (3H, doublet, J = 7.3 Hz);
   1.30 (3H, doublet, J = 6.4 Hz);
   1.64 (1H, doubled doublet of doublets, J = 13.7, 6.8 & 5.4 Hz);
   2.74 (1H, doublet of triplets, J = 13.7 & 8.8 Hz);
   3.07 (1H, doublet of doublets, J = 12.2 & 3.4 Hz);
   3.17 (1H, doublet of doublets, J = 12.2 & 5.4 Hz); 3.34 - 3.47 (2H, multiplet);
   3.54 - 3.90 (9H, multiplet);
   4.13 (1H, doublet of doublets, J = 8.8 & 6.8 Hz);
   4.19 - 4.13 (2H, multiplet).
Nuclear Magnetic Resonance Spectrum (¹³C, D₂O, external standard: tetramethylsilane) δ ppm:
   16.0, 20.2, 35.5, 41.3, 42.6, 42.8, 43.5, 44.2, 44.7, 53.9, 56.0, 57.4, 58.4, 65.2, 132.0, 140.9, 156.6, 167.8, 172.5, 176.4.

### EXAMPLE 13

### (1R,5S,6S)-2-[(2S,4S)-2-(4-Amidinopiperazin-1-yl-carbonyl)-1-methylpyrrolidin-4-ylthio]-6-[(1R)-1-hydroxyethyl]-1-methyl-1-carbapen-2-em-3-carboxylic acid

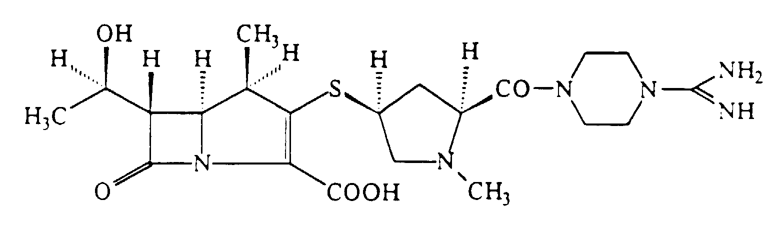

### 13(1) 4-Nitrobenzyl (1R,5S,6S)-6-[(1R)-1-hydroxyethyl]-1-methyl-2-{(2S,4S)-1-methyl-2-[4-(4-nitrobenzyloxycarbonylamidino)piperazin-1-ylcarbonyl]pyrrolidin-4-yl-thio}-1-carbapen-2-em-3-carboxylate

1600 mg of 4-nitrobenzyl (1R,5R,6S)-2-(diphenylphosphoryloxy)-6-[(1R)-1-hydroxyethyl]-1-methyl-1-carbapen-2-em-3-carboxylate (prepared as described in Preparation 32) were dissolved in 16 ml of dry acetonitrile, and a solution of 1120 mg of (2S,4S)-4-mercapto-1-methyl-2-[4-(4-nitrobenzyloxycarbonylamidino)piperazin-1-ylcarbonyl]pyrrolidine (prepared as described in Preparation 2) in 11 ml of dry acetonitrile and 430 µℓ of diisopropylethylamine were added dropwise to the resulting solution, whilst ice-cooling. The resulting mixture was stirred at the same temperature overnight, after which it was concentrated by evaporation under reduced pressure. The resulting residue was diluted with ethyl acetate, and the mixture was washed with an aqueous solution of sodium hydrogencarbonate, with water and with an aqueous solution of sodium chloride, in that order. The ethyl acetate layer was dehydrated over anhydrous sodium sulphate and then concentrated by evaporation under reduced pressure. The resulting residue was subjected to reverse phase silica gel column chromatography (Cosmosil 75C₁₈-prep, manufactured by Nacalai Tesque, 300 ml), eluted with a 1 : 1 by volume mixture of acetonitrile and water. The fractions containing the desired compound were collected and concentrated by evaporation under reduced pressure, to obtain 1520 mg of the title compound, as a powder.
Infrared Absorption Spectrum (KBr), νₘₐₓ cm⁻¹:
   1771, 1708, 1648, 1606, 1544, 1521, 1448, 1347.
Nuclear Magnetic Resonance Spectrum (hexadeuterated dimethyl sulphoxide, 270 MHz) δ ppm:
   1.10 - 1.20 (6H, multiplet);
   1.58 - 1.69 (1H, multiplet);
   2.24 (3H, singlet);
   2.60 - 2.80 (2H, multiplet);
   2.95 - 3.02 (1H, multiplet);
   3.20 - 3.88 (12H, multiplet);
   3.90 - 4.05 (1H, multiplet);
   4.21 (1H, doublet of doublets, J = 9.3 & 2.4 Hz);
   5.05 (1H, doublet, J = 5.4 Hz);
   5.13 (2H, singlet);
   5.29 (2H, doublet, J = 13.7 Hz);
   5.45 (2H, doublet, J = 13.7 Hz);
   7.59 (2H, doublet, J = 8.8 Hz);
   7.73 (2H, doublet, J = 8.8 Hz);
   7.90-8.20 (2H, broad);
   8.21 (2H, doublet, J = 8.8 Hz);
   8.23 (2H, doublet, J = 8.8 Hz).

### 13(2) (1R,5S,6S)-2-[(2S,4S)-2-(4-Amidinopiperazin-1-yl-carbonyl)-1-methylpyrrolidin-4-ylthio]-6-[(1R)-1-hydroxyethyl]-1-methyl-1-carbapen-2-em-3-carboxylic acid

1500 mg of 4-nitrobenzyl (1R,5S,6S)-6-[(1R)-1-hydroxyethyl]-1-methyl-2-{(2S,4S)-1-methyl-2-[4-(4-nitrobenzyloxycarbonylamidino)piperazin-1-ylcarbonyl]pyrrolidin-4-yl-thio}-1-carbapen-2-em-3-carboxylate [prepared as described in step (1) above] were dissolved in 75 ml of a 1 : 1 by volume mixture of tetrahydrofuran and water, and 2400 mg of a 10% w/w palladium-on-carbon catalyst were added to the resulting solution. The mixture was then hydrogenated in an atmosphere of hydrogen at 28°C for 1 hour. At the end of this time, the catalyst was removed by filtration, and the filtrate was washed with diethyl ether. The filtrate was then concentrated to 20 ml by evaporation under reduced pressure. The solution was then subjected to reverse phase silica gel column chromatography (Cosmosil 75C₁₈-prep, manufactured by Nacalai Tesque, 100 ml), eluted with mixtures of acetonitrile and water in proportions of 0 : 100, 2 : 98, 4 : 96, 6 : 94 and 8 : 92 by volume, in that order. The fractions containing the desired compound were collected, concentrated to 3 ml by evaporation under reduced pressure, and cooled to precipitate colourless needle-like crystals. The crystals were filtered off and dried to obtain 435 mg of the title compound as crystals, melting at 218 - 220°C (with decomposition).
Ultraviolet Absorption Spectrum (H₂O), λₘₐₓ nm:
   298.
Infrared Absorption Spectrum (KBr), νₘₐₓ cm⁻¹:
   1755, 1652, 1606, 1449, 1386, 1246.
Nuclear Magnetic Resonance Spectrum (270MHz, D₂O, internal standard: tetradeuterated sodium trimethylsilylpropionate) δ ppm:
   1.20 (3H, doublet, J = 6.8 Hz);
   1.30 (3H, doublet, J = 6.3 Hz);
   1.66 (1H, doubled doublet of doublets, J = 13.7, 8.8 & 5.4 Hz);
   2.28 (3H, singlet);
   2.74 - 2.87 (2H, multiplet);
   3.09 (1H, doublet of doublets, J = 10.8 & 1.5 Hz);
   3.30 - 3.90 (12H, multiplet);
   4.16 - 4.31 (2H, multiplet).
Nuclear Magnetic Resonance Spectrum (¹³C, D₂O, external standard: tetramethylsilane) δ ppm:
   15.7, 19.9, 34.9, 39.1, 39.3, 40.9, 42.4, 43.3, 43.9, 44.5, 55.7, 58.0, 62.2, 64.9, 65.0, 131.2, 141.6, 156.3, 167.5, 171.1, 175.9.

### EXAMPLE 14

### (1R,5S,6S)-2-[(2S,4S)-2-(4-Amidinohomopiperazin-1-yl-carbonyl)pyrrolidin-4-ylthio]-6-[(1R)-1-hydroxyethyl]-1-methyl-1-carbapen-2-em-3-carboxylic acid

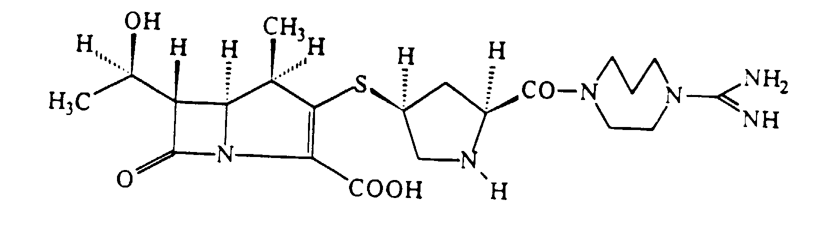

A procedure similar to that described in Example 13 was repeated, but using 4-nitrobenzyl (1R,5R,6S)-2-(diphenylphosphoryloxy)-6-[(1R)-1-hydroxyethyl]-1-methyl-1-carbapen-2-em-3-carboxylate (prepared as described in Preparation 32) and (2S,4S)-4-mercapto-1-(4-nitrobenzyloxycarbonyl)-2-[4-(4-nitrobenzyloxycarbonylamidino)homopiperazin-1-ylcarbonyl]pyrrolidine (prepared as described in Preparation 3) as starting materials, in relative proportions similar to those used in that Example, to obtain the title compound.
Ultraviolet Absorption Spectrum (H₂O), λₘₐₓ nm:
   299.
Infrared Absorption Spectrum (KBr), νₘₐₓ cm⁻¹:
   1754, 1608, 1580, 1456, 1387, 1262.
Nuclear Magnetic Resonance Spectrum (¹H, 270MHz, D₂O, internal standard: tetradeuterated sodium trimethylsilylpropionate) δ ppm:
   1.21 (3H, doublet, J = 7.2 Hz);
   1.30 (3H, doublet, J = 6.3 Hz);
   1.46 - 1.54 (1H, multiplet);
   1.84 - 1.96 (2H, multiplet);
   2.70 - 2.80 (1H, multiplet);
   3.08 (1H, doublet of doublets, J = 12.4 & 3.3 Hz);
   3.14 (1H, doublet of doublets, J = 12.4 & 5.4 Hz);
   3.37 - 3.45 (2H, multiplet);
   3.52 - 3.95 (9H, multiplet);
   4.04 - 4.14 (1H, multiplet);
   4.20 - 4.29 (2H, multiplet).

### EXAMPLE 15

### (1R,5S,6S)-2-[(2S,4S)-2-(4-Amidinohomopiperazin-1-yl-carbonyl)-1-methylpyrrolidin-4-ylthio]-6-[(1R)-1-hydroxyethyl]-1-methyl-1-carbapen-2-em-3-carboxylic acid

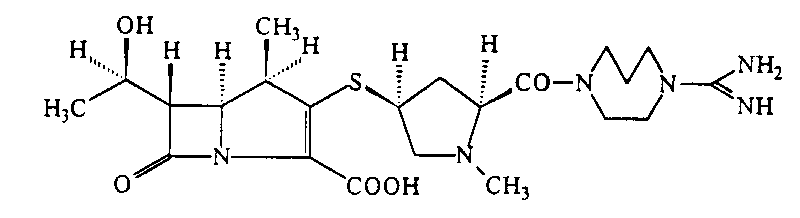

A procedure similar to that described in Example 13 was repeated, but using 4-nitrobenzyl (1R,5R,6S)-2-(diphenylphosphoryloxy)-6-[(1R)-1-hydroxyethyl]-1-methyl-1-carbapen-2-em-3-carboxylate (prepared as described in Preparation 32) and (2S,4S)-4-mercapto-1-methyl-2-[4-(4-nitrobenzyloxycarbonylamidino)homopiperazin-1-ylcarbonyl]pyrrolidine (prepared as described in Preparation 4) as starting materials, in relative proportions similar to those used in that Example, to obtain the title compound.
Ultraviolet Absorption Spectrum (H₂O), λₘₐₓ nm:
   298.
Infrared Absorption Spectrum (KBr), νₘₐₓ cm⁻¹:
   1755, 1650, 1607, 1455, 1385, 1258.
Nuclear Magnetic Resonance Spectrum (270MHz, D₂O, internal standard: tetradeuterated sodium trimethylsilylpropionate) δ ppm:
   1.20 (3H, doublet, J = 7.2 Hz);
   1.30 (3H, doublet, J = 6.3 Hz);
   1.60 (1H, doubled doublet of doublets, J = 13.5, 9.0 & 5.5 Hz);
   1.80 - 1.95 (2H, multiplet);
   2.24 & 2.25 (together 3H, two singlets);
   2.74 - 2.87 (2H, multiplet);
   3.09 (1H, doublet, J = 9.0 Hz);
   3.33 - 3.95 (12H, multiplet);
   4.19 (1H, doublet of doublets, J = 9.0 & 2.4 Hz);
   4.22 - 4.28 (1H, multiplet).

### EXAMPLE 16

### (1R,5S,6S)-2-[(2S,4S)-2-(4-Guanidinopiperidin-1-yl-carbonyl)pyrrolidin-4-ylthio]-6-[(1R)-1-hydroxyethyl]-1-methyl-1-carbapen-2-em-3-carboxylic acid

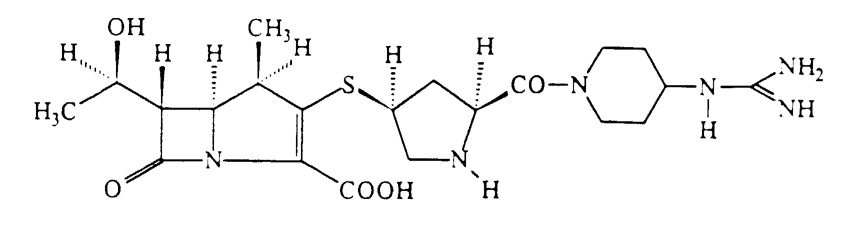

A procedure similar to that described in Example 13 was repeated, but using 4-nitrobenzyl (1R,5R,6S)-2-(diphenylphosphoryloxy)-6-[(1R)-1-hydroxyethyl]-1-methyl-l-carbapen-2-em-3-carboxylate (prepared as described in Preparation 32) and (2S,4S)-4-mercapto-1-(4-nitrobenzyloxycarbonyl)-2-[4-(4-nitrobenzyloxycarbonylguanidino)piperidin-1-ylcarbonyl]pyrrolidine (prepared as described in Preparation 5) as starting materials, in relative proportions similar to those used in that Example, to obtain the title compound.
Ultraviolet Absorption Spectrum (H₂O), λₘₐₓ nm:
299.

### EXAMPLE 17

### (1R,5S,6S)-2-[(2S,4S)-2-(4-Guanidinopiperidin-1-yl-carbonyl)-1-methylpyrrolidin-4-ylthio]-6-[(1R)-1-hydroxyethyl]-1-methyl-1-carbapen-2-em-3-carboxylic acid

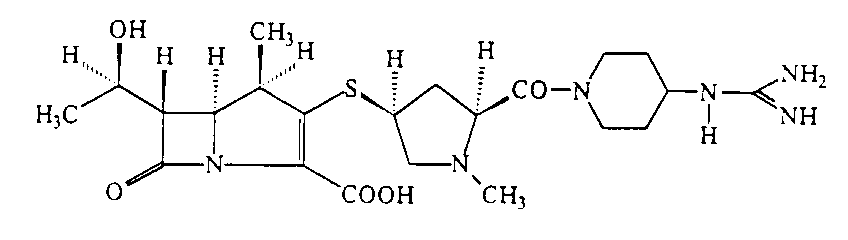

A procedure similar to that described in Example 13 was repeated, but using 4-nitrobenzyl (1R,5R,6S)-2-(diphenylphosphoryloxy)-6-[1(R)-1-hydroxyethyl]-1-methyl-1-carbapen-2-em-3-carboxylate (prepared as described in Preparation 32) and (2S,4S)-4-mercapto-1-methyl-2-[4-(4-nitrobenzyloxycarbonylguanidino)piperidin-1-ylcarbonyl]pyrrolidine (prepared as described in Preparation 6) as starting materials, in relative proportions similar to those used in that Example, to obtain the title compound.
Ultraviolet Absorption Spectrum (H₂O), λₘₐₓ nm:
298.

### EXAMPLE 18

### (1R,5S,6S)-2-[(2S,4S)-2-[(3S)-3-Guanidinopyrrolidin-1-ylcarbonyl)pyrrolidin-4-ylthio]-6-[(1R)-1-hydroxyethyl]-1-methyl-1-carbapen-2-em-3-carboxylic acid

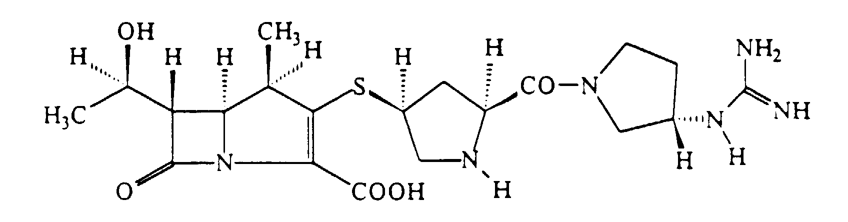

A procedure similar to that described in Example 13 was repeated, but using 4-nitrobenzyl (1R,5R,6S)-2-(diphenylphosphoryloxy)-6-[(1R)-1-hydroxyethyl]-1-methyl-1-carbapen-2-em-3-carboxylate (prepared as described in Preparation 32) and (2S,4S)-4-mercapto-1-(4-nitrobenzyloxycarbonyl)-2-[(3S)-3-(4-nitrobenzyloxycarbonylguanidino)pyrrolidin-1-ylcarbonyl]pyrrolidine (prepared as described in Preparation 7) as starting materials, in relative proportions similar to those used in that Example, to obtain the title compound.
Ultraviolet Absorption Spectrum (H₂O), λₘₐₓ nm:
299.

### EXAMPLE 19

### (1R,5S,6S)-2-[(2S,4S)-2-[(3S)-3-Guanidinopyrrolidin-1-ylcarbonyl)-1-methylpyrrolidin-4-ylthio]-6-[(1R)-1-hydroxyethyl]-1-methyl-1-carbapen-2-em-3-carboxylic acid

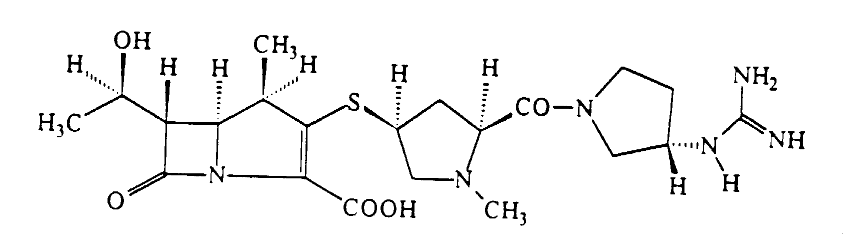

A procedure similar to that described in Example 13 was repeated, but using 4-nitrobenzyl (1R,5R,6S)-2-(diphenylphosphoryloxy)-6-[(1R)-1-hydroxyethyl]-1-methyl-1-carbapen-2-em-3-carboxylate (prepared as described in Preparation 32) and (2S,4S)-4-mercapto-1-methyl-2-[(3S)-3-(4-nitrobenzyloxycarbonylguanidino)pyrrolidin-1-yl-carbonyl]pyrrolidine (prepared as described in Preparation 8) as starting materials, in relative proportions similar to those used in that Example, to obtain the title compound.
Ultraviolet Absorption Spectrum (H₂O), λₘₐₓ nm:
298.

### EXAMPLE 20

### (1R,5S,6S)-2-[(2S,4S)-2-(3-Guanidinoazetidin-1-ylcarbonyl)pyrrolidin-4-ylthio]-6-[(1R)-1-hydroxyethyl]-1-methyl-1-carbapen-2-em-3-carboxylic acid

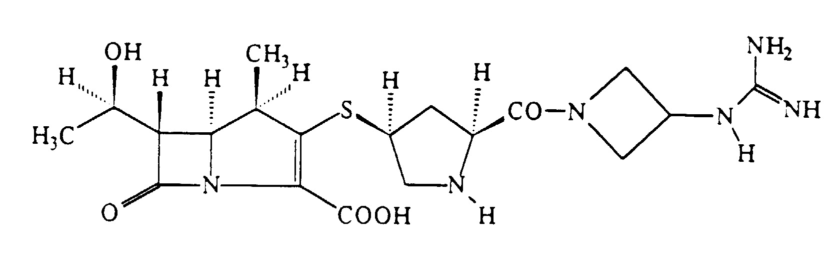

A procedure similar to that described in Example 13 was repeated, but using 4-nitrobenzyl (1R,5R,6S)-2-(diphenylphosphoryloxy)-6-[(1R)-1-hydroxyethyl]-1-methyl-1-carbapen-2-em-3-carboxylate (prepared as described in Preparation 32) and (2S,4S)-4-mercapto-1-(4-nitrobenzyloxycarbonyl)-2-[3-(4-nitrobenzyloxycarbonylguanidino)azetidin-1-ylcarbonyl]pyrrolidine (prepared as described in Preparation 9) as starting materials, in relative proportions similar to those used in that Example, to obtain the title compound.
Ultraviolet Absorption Spectrum (H₂O), λₘₐₓ nm:
299.

### EXAMPLE 21

### (1R,5S,6S)-2-[(2S,4S)-2-(3-Guanidinoazetidin-1-ylcarbonyl)-1-methylpyrrolidin-4-ylthio]-6-[(1R)-1-hydroxyethyl]-1-methyl-1-carbapen-2-em-3-carboxylic acid

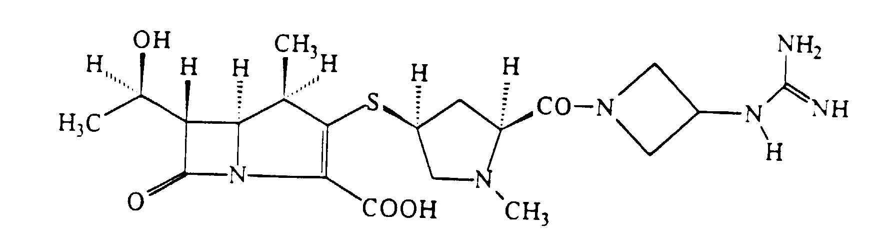

A procedure similar to that described in Example 13 was repeated, but using 4-nitrobenzyl (1R,5R,6S)-2-(diphenylphosphoryloxy)-6-[(1R)-1-hydroxyethyl]-1-methyl-1-carbapen-2-em-3-carboxylate (prepared as described in Preparation 32) and (2S,4S)-4-mercapto-1-methyl-2-[3-(4-nitrobenzyloxycarbonylguanidino)azetidin-1-ylcarbonyl]pyrrolidine (prepared as described in Preparation 10) as starting materials, in relative proportions similar to those used in that Example, to obtain the title compound.
Ultraviolet Absorption Spectrum (H₂O), λₘₐₓ nm:
298.

### EXAMPLE 24

### (1R,5S,6S)-6-[(1R)-1-Hydroxyethyl]-1-methyl-2-{(2S,4S)-2-[4-(methylamidino)piperazin-1-ylcarbonyl]pyrrolidin4-ylthio}-1-carbapen-2-em-3-carboxylic acid

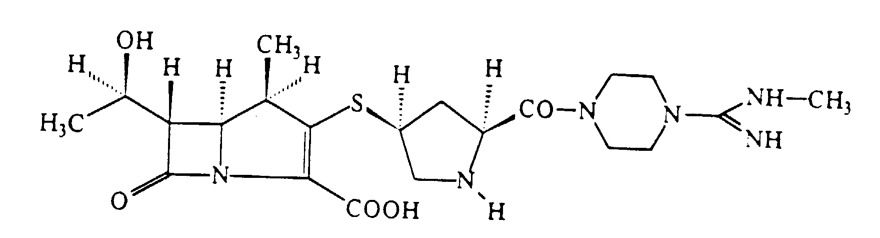

A procedure similar to that described in Example 13 was repeated, but using 4-nitrobenzyl (1R,5R,6S)-2-(diphenylphosphoryloxy)-6-[(1R)-1-hydroxyethyl]-1-methyl-1-carbapen-2-em-3-carboxylate (prepared as described in Preparation 32) and (2S,4S)-4-mercapto-2-[4-(methyl-4-nitrobenzyloxycarbonylamidino)piperazin-1-ylcarbonyl]-1-(4-nitrobenzyloxycarbonyl)pyrrolidine (prepared as described in Preparation 13) as starting materials, in relative proportions similar to those used in that Example, to obtain the title compound.
Ultraviolet Absorption Spectrum (H₂O), λₘₐₓ nm:
299.

### EXAMPLE 25

### (1R,5S,6S)-6-[(1R)-1-Hydroxyethyl]-1-methyl-2-{(2S,4S)-1-methyl-2-[4-(methylamidino)piperazin-1-ylcarbonyl]pyrrolidin-4-ylthio}-1-carbapen-2-em-3-carboxylic acid

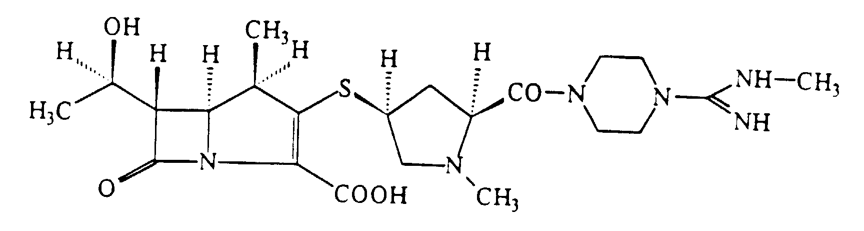

A procedure similar to that described in Example 13 was repeated, but using 4-nitrobenzyl (1R,5R,6S)-2-(diphenylphosphoryloxy)-6-[(1R)-1-hydroxyethyl]-1-methyl-1-carbapen-2-em-3-carboxylate (prepared as described in Preparation 32) and (2S,4S)-4-mercapto-1-methyl-2-[4-(methyl-4-nitrobenzyloxycarbonylamidino)piperazin-1-yl-carbonyl]pyrrolidine (prepared as described in Preparation 14) as starting materials, in relative proportions similar to those used in that Example, to obtain the title compound.
Ultraviolet Absorption Spectrum (H₂O), λₘₐₓ nm:
298.

### EXAMPLE 26

### (1R,5S,6S)-2-{(2S,4S)-2-[(3R)-3-Guanidinopyrrolidin1-ylcarbonyl]pyrrolidin-4-ylthio]-6-[(1R)-1-hydroxyethyl]-1-methyl-1-carbapen-2-em-3-carboxylic acid

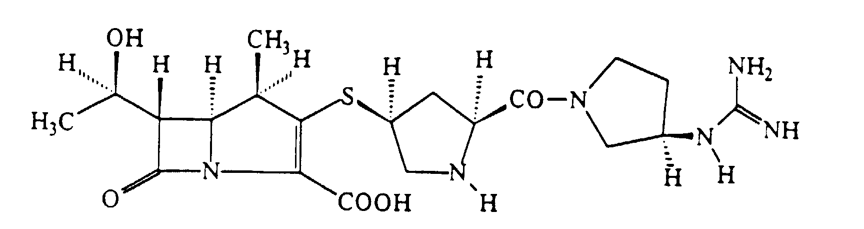

A procedure similar to that described in Example 13 was repeated, but using 4-nitrobenzyl (1R,5R,6S)-2-(diphenylphosphoryloxy)-6-1(1R)-1-hydroxyethyl]-1-methyl-1-carbapen-2-em-3-carboxylate (prepared as described in Preparation 32) and (2S,4S)-4-mercapto-1-(4-nitrobenzyloxycarbonyl)-2-[(3R)-3-(4-nitrobenzyloxycarbonylguanidino)pyrrolidin-1-ylcarbonyl]pyrrolidine (prepared as described in Preparation 15) as starting materials, in relative proportions similar to those used in that Example, to obtain the title compound.
Ultraviolet Absorption Spectrum (H₂O), λₘₐₓ nm:
299.

### EXAMPLE 27

### (1R,5S,6S)-2-{(2S,4S)-2-[(3R)-3-Guanidinopyrrolidin-1-ylcarbonyl]-1-methylpyrrolidin-4-ylthio}-6-[(1R)-1-hydroxyethyl]-1-methyl-1-carbapen-2-em-3-carboxylic acid

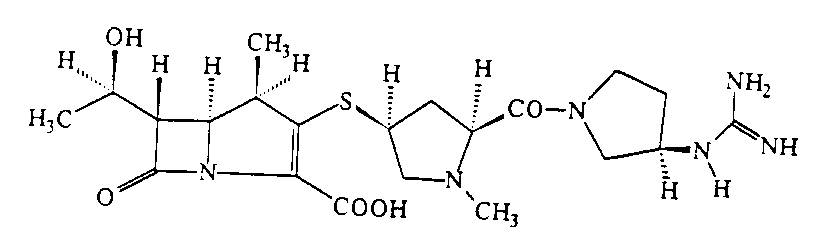

A procedure similar to that described in Example 13 was repeated, but using 4-nitrobenzyl (1R,5R,6S)-2-(diphenylphosphoryloxy)-6-[1(R)-1-hydroxyethyl]-1-methyl-1-carbapen-2-em-3-carboxylate (prepared as described in Preparation 32) and (2S,4S)-4-mercapto-1-methyl-2-[(3R)-3-(4-nitrobenzyloxycarbonylguanidino)pyrrolidin-1-yl-carbonyl]pyrrolidine (prepared as described in Preparation 16) as starting materials, in relative proportions similar to those used in that Example, to obtain the title compound.
Ultraviolet Absorption Spectrum (H₂O), λₘₐₓ nm:
298.

### EXAMPLE 28

### (1R,5S,6S)-2-{(2S,4S)-2-[(3R)-4-Amidino-3-methylpiperazin-1-ylcarbonyl]pyrrolidin-4-ylthio}-6-[(1R)-1-hydroxyethyl]-1-methyl-1-carbapen-2-em-3-carboxylic acid

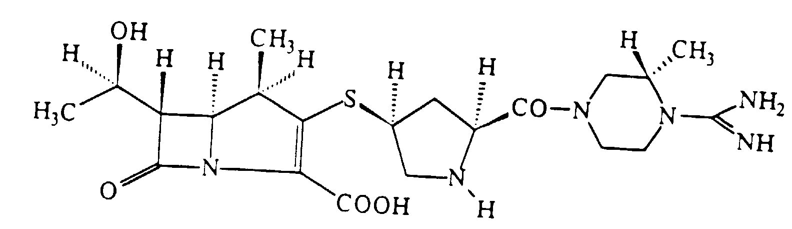

A procedure similar to that described in Example 13 was repeated, but using 4-nitrobenzyl (1R,5R,6S)-2-(diphenylphosphoryloxy)-6-[(1R)-1-hydroxyethyl]-1-methyl-1-carbapen-2-em-3-carboxylate (prepared as described in Preparation 32) and (2S,4S)-4-mercapto-1-(4-nitrobenzyloxycarbonyl)-2-[(3R)-4-(4-nitrobenzyloxycarbonylamidino)-3-methylpiperazin-1-ylcarbonyl]pyrrolidine (prepared as described in Preparation 17) as starting materials, in relative proportions similar to those used in that Example, to obtain the title compound.
Ultraviolet Absorption Spectrum (H₂O), λₘₐₓ nm:
299.

### EXAMPLE 29

### (1R,5S,6S)-2-{(2S,4S)-2-[(3R)-4-Amidino-3-methylpiperazin-1-ylcarbonyl]-1-methylpyrrolidin-4-ylthio}-6-[(1R)-1-hydroxyethyl]-1-methyl-1-carbapen-2-em-3-carboxylic acid

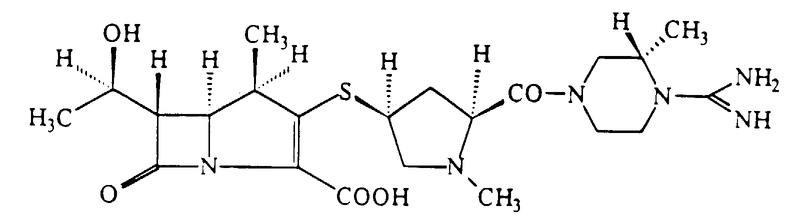

A procedure similar to that described in Example 13 was repeated, but using 4-nitrobenzyl (1R,5R,6S)-2-(diphenylphosphoryloxy)-6-[(1R)-1-hydroxyethyl]-1-methyl-1-carbapen-2-em-3-carboxylate (prepared as described in Preparation 32) and (2S,4S)-4-mercapto-l-methyl-2-[(3R)-4-(4-nitrobenzyloxycarbonylamidino)-3-methylpiperazin-1-ylcarbonyl]pyrrolidine (prepared as described in Preparation 18) as starting materials, in relative proportions similar to those used in that Example, to obtain the title compound.
Ultraviolet Absorption Spectrum (H₂O), λₘₐₓ nm:
298.

### PREPARATION 1

### (2S,4S)-4-Mercapto-2-[4-(4-nitrobenzyloxycarbonylamidino)piperazin-1-ylcarbonyl]-1-(4-nitrobenzyloxycarbonyl)pyrrolidine trifluoromethanesulphonate

### 1(a) 1-Amidino-4-t-butoxycarbonylpiperazine hemisulphate

2.50 g of 1-amidinopiperazine hemisulphate were dissolved in 60 ml of a 1 : 1 by volume mixture of tetrahydrofuran and water, and a solution of 3.40 g of di-t-butyl dicarbonate in 10 ml of tetrahydrofuran was added to the resulting solution, after which the mixture was stirred at room temperature for 2.5 hours. At the end of this time, the tetrahydrofuran was removed from the reaction mixture by distillation under reduced pressure, the insolubles were filtered off and the filtrate was evaporated to dryness under reduced pressure. The resulting residue was extracted with ethanol (twice, each time with 50 ml) and methanol (twice, each time with 50 ml). The extract was evaporated to dryness to obtain 2.48 g of the title compound, as crystals, melting at 278°C (with decomposition).
Nuclear Magnetic Resonance Spectrum (270MHz, D₂O, internal standard substance: tetradeuterated sodium trimethylsilylpropionate) δ ppm:
   1.47 (9H, singlet);
   3.49 - 3.64 (8H, multiplet).
Infrared Absorption Spectrum (KBr), νₘₐₓ cm⁻¹:
   1696, 1656, 1616, 1416, 1169, 1121.

### 1(b) 4-t-Butoxycarbonyl-1-(4-nitrobenzyloxycarbonylamidino)piperazine

2.22 g of 1-amidino-4-t-butoxycarbonylpiperazine hemisulphate [prepared as described in step (a) above] were dissolved in 90 ml of a 1 : 1 by volume mixture of tetrahydrofuran and water, and a solution of 1.89 g of 4-nitrobenzyloxycarbonyl chloride in 16 ml of tetrahydrofuran and 16 ml of a 1N aqueous solution of sodium hydroxide were added thereto at the same time, whilst stirring and ice-cooling. The resulting mixture was stirred for 30 minutes under the same conditions, and then the organic solvent was removed from the reaction mixture by distillation under reduced pressure. The residual aqueous solution was extracted with ethyl acetate, and the ethyl acetate extract was washed with an aqueous solution of sodium chloride and dried over anhydrous sodium sulphate. The mixture was then concentrated by evaporation under reduced pressure. The resulting residue was subjected to silica gel column chromatography, eluted with a 4 : 1 by volume mixture of ethyl acetate and hexane. The fraction containing the desired compound was concentrated by evaporation under reduced pressure, to obtain 2.32 g of the title compound, as an amorphous powder.
Nuclear Magnetic Resonance Spectrum (270MHz, CDCl₃) δ ppm:
   1.47 (9H, singlet);
   3.45 - 3.61 (8H, multiplet);
   5.21 (2H, singlet);
   6.90 - 7.20 (2H, broad);
   7.56 (2H, doublet, J = 8.6 Hz);
   8.20 (2H, doublet, J = 8.6 Hz).
Infrared Absorption Spectrum (KBr), ν_{maX} cm⁻¹:
   1698, 1652, 1601, 1547, 1523, 1279.

### 1(c) 1-(4-Nitrobenzyloxycarbonylamidino)piperazine

750 mg of 4-t-butoxycarbonyl-1-(4-nitrobenzyloxycarbonylamidino)piperazine [prepared as described in step (b) above] were dissolved in 10 ml of trifluoroacetic acid, and the resulting solution was stirred at room temperature for 30 minutes. At the end of this time, the reaction mixture was concentrated by evaporation under reduced pressure, and the resulting residue was dissolved in a mixture of ethyl acetate and water, and an aqueous solution of sodium hydrogencarbonate was added thereto to make the mixture alkaline. The ethyl acetate layer was separated and the aqueous layer was extracted with ethyl acetate three times. The ethyl acetate layer and all of the ethyl acetate washings were combined and concentrated by evaporation under reduced pressure, to obtain 530 mg of the title compound, as a powder.
Nuclear Magnetic Resonance Spectrum (270MHz, D₂O, internal standard: tetradeuterated sodium trimethylsilylpropionate) δ ppm:
3.27 (4H, triplet, J = 5.3 Hz);
3.80 (4H, triplet, J = 5.3 Hz);
5.25 (2H, singlet);
7.61 (2H, doublet, J = 8.6 Hz);
8.27 (2H, doublet, J = 8.6Hz).

### 1(d) (2S,4S)-4-(4-Methoxybenzylthio)-2-[4-(4-nitrobenzyloxycarbonylamidino)piperazin-1-ylcarbonyl]-1-(4-nitrobenzyloxycarbonyl)pyrrolidine

740 mg of (2S,4S)-4-(4-methoxybenzylthio)-1-(4-nitrobenzyloxy)pyrrolidine-2-carboxylic acid was dissolved in 7.4 ml of dry acetonitrile, and 330 mg of N,N'-carbonyldiimidazole were added to the resulting solution, after which the mixture was stirred at room temperature for 30 minutes. At the end of this time, a solution of 525 mg of 1-(4-nitrobenzyloxycarbonylamidino)piperazine [prepared as described in step (c) above] dissolved in 10 ml of acetonitrile was added to the resulting mixture, and the mixture was left to stand overnight under the same conditions. The reaction mixture was then diluted with ethyl acetate, after which it was washed with an aqueous solution of sodium hydrogencarbonate, with water and with an aqueous solution of sodium chloride, in that order, dried over anhydrous sodium sulphate and concentrated by evaporation under reduced pressure. The resulting residue was subjected to silica gel column chromatography, eluted first with ethyl acetate alone and then with a 4 : 1 by volume mixture of ethyl acetate and acetonitrile, in that order. The fractions containing the desired compound were concentrated by evaporation under reduced pressure, to obtain 890 mg of the title compound, as a powder.
Infrared Absorption Spectrum (KBr), νₘₐₓ cm⁻¹:
   1709, 1652, 1608, 1520, 1439, 1346.
Nuclear Magnetic Resonance Spectrum (270MHz, CDCl₃ + D₂O) δ ppm:
   1.72 - 1.89 (1H, multiplet);
   2.39 - 2.52 (1H, multiplet);
   3.03 - 3.18 (1H, multiplet);
   3.30 - 4.10 (15H, multiplet);
   4.51 - 4.62 (1H, multiplet);
   4.98 - 5.32 (4H, multiplet);
   6.85 (2H, doublet, J = 8.8 Hz);
   7.23 (2H, doublet, J = 8.8 Hz);
   7.40 - 7.58 (4H, multiplet);
   8.15 - 8.25 (4H, multiplet).

### 1(e) (2S,4S)-4-Mercapto-2-[4-(4-nitrobenzyloxycarbonylamidino)piperazin-1-ylcarbonyl]-1-(4-nitrobenzyloxycarbonyl)pyrrolidine trifluoromethanesulphonate

880 mg of (2S,4S)-4-(4-methoxybenzylthio)-2-[4-(4-nitrobenzyloxycarbonylamidino)piperazin-1-ylcarbonyl]-1-(4-nitrobenzyloxycarbonyl)pyrrolidine [prepared as described in step (d) above] were dissolved in a mixture of 4.4 ml of trifluoroacetic acid and 0.88 ml of anisole, and then 160 µℓ of trifluoromethanesulphonic acid were added dropwise to this solution, whilst stirring and ice-cooling. The resulting mixture was then stirred at room temperature for 3 hours, after which it was concentrated by evaporation under reduced pressure. The resulting residue was washed three times with diethyl ether, and the powder thus formed was dried to obtain 918 mg of the title compound, as a powder.
Infrared Absorption Spectrum (KBr), νₘₐₓ cm⁻¹:
   1761, 1671, 1618, 1523, 1443, 1348, 1285, 1246, 1225, 1169.
Nuclear Magnetic Resonance Spectrum (hexadeuterated dimethyl sulphoxide + D₂O, 270 MHz) δ ppm:
   1.60 - 1.76 (1H, multiplet);
   2.65 - 2.82 (1H, multiplet);
   3.05 - 3.80 (10H, multiplet);
   3.94 & 4.05 (together 1H, two doublets of doublets, J = 9.8 and 6.8 Hz);
   4.74 & 4.83 (together 1H, two triplets, J = 7.8 Hz); 5.05 - 5.25 (2H, multiplet);
   5.40 (2H, singlet);
   7.53 & 7.63 (together 2H, two doublets, J = 8.8 Hz);
   7.72 (2H, doublet, J = 8.8 Hz);
   8.21 & 8.23 (together 2H, two doublets, J = 8.8 Hz);
   8.28 (2H, doublet, J = 8.8Hz).

### PREPARATION 2

### (2S,4S)-4-Mercapto-1-methyl-2-[4-(4-nitrobenzyloxycarbonylamidino)piperazin-1-ylcarbonyl]pyrrolidine

### 2(a) 1-(4-Nitrobenzyloxycarbonylamidino)piperazine bis(trifluoroacetate)

750 mg of 4-t-butoxycarbonyl-1-(4-nitrobenzyloxycarbonylamidino)piperazine [prepared as described in Preparation 1(b)] were dissolved in 12 ml of trifluoroacetic acid, and the resulting solution was stirred at room temperature for 30 minutes. At the end of this time, the reaction mixture was concentrated by evaporation under reduced pressure, and diethyl ether was added to the residue. The powder thus formed was washed three times with diethyl ether and then dried to obtain 1010 mg of the title compound, as a colourless powder.
Infrared Absorption Spectrum (KBr), νₘₐₓ cm⁻¹:
   1771, 1698, 1665, 1623, 1518, 1353, 1221, 1197.
Nuclear Magnetic Resonance Spectrum (hexadeuterated dimethyl sulphoxide + D₂O, 270 MHz) δ ppm:
   3.21 (4H, triplet, J = 5.4 Hz);
   3.73 (4H, triplet, J = 5.4 Hz);
   5.31 (2H, singlet);
   7.68 (2H, doublet, J = 8.8 Hz);
   8.26 (2H, doublet, J = 8.8Hz).

### 2(b) (2S,4S)-4-(4-Methoxybenzylthio)-1-methyl-2-[4-(4-nitrobenzyloxycarbonylamidino)piperazin-1-ylcarbonyl]pyrrolidine

480 mg of (2S,4S)-4-(4-methoxybenzylthio)-1-methylpyrrolidine-2-carboxylic acid were suspended in 20 ml of dry acetonitrile, and 325 mg of N,N'-carbonyldiimidazole were added to this suspension, after which the mixture was stirred at 40°C for 1 hour. At the end of this time, the reaction mixture was cooled with ice, and 980 mg of 1-(4-nitrobenzyloxycarbonylamidino)piperazine bis(trifluoroacetate) [prepared as described in step (a) above] and 320 µℓ of diisopropylethylamine were added to the mixture, which was then stirred at room temperature overnight. At the end of this time, the reaction mixture was diluted with ethyl acetate and washed with an aqueous solution of sodium hydrogencarbonate, with water (4 times) and with an aqueous solution of sodium chloride, in that order. The ethyl acetate solution was dried over anhydrous sodium sulphate and then concentrated by evaporation under reduced pressure. The resulting residue was subjected to silica gel column chromatography, eluted with a 4 : 1 by volume mixture of ethyl acetate and methanol. The fraction containing the desired compound was concentrated by evaporation under reduced pressure, to obtain 860 mg of the title compound, as a powder.
Infrared Absorption Spectrum (KBr), νₘₐₓ cm⁻¹:
   1648, 1609, 1542, 1513, 1440, 1346, 1303, 1273, 1239.
Nuclear Magnetic Resonance Spectrum (270MHz, CDCl₃ + D₂O) δ ppm:
   1.78 (1H, doubled doublet of doublets, J = 13.7, 9.3 & 5.4 Hz);
   2.33 (3H, singlet);
   2.48 - 2.60 (2H, multiplet);
   3.03 - 3.24 (3H, multiplet);
   3.70 (2H, singlet);
   3.80 (3H, singlet);
   3.45 - 4.15 (8H, multiplet);
   5.21 (2H, singlet);
   6,84 (2H, doublet, J = 8.8 Hz);
   7.20 (2H, doublet, J = 8.8 Hz);
   7.56 (2H, doublet, J = 8.8 Hz);
   8.20 (2H, doublet, J = 8.8 Hz).

### 2(c) (2S,4S)-4-Mercapto-1-methyl-2-[4-(4-nitrobenzyloxycarbonylamidino)piperazin-1-ylcarbonyl]pyrrolidine

1450 mg of (2S,4S)-4-(4-methoxybenzylthio)-1-methyl-2-[4-(4-nitrobenzyloxycarbonylamidino)piperazin-1-yl-carbonyl]pyrrolidine [prepared as described in step (b) above] were dissolved in a mixture of 7.25 ml of trifluoroacetic acid and 1.45 ml of anisole, and then 562 µℓ of trifluoromethanesulphonic acid were added dropwise, whilst stirring and ice-cooling, to the resulting solution. The reaction mixture was stirred for 60 minutes under the same conditions and then at room temperature for 30 minutes, after which it was concentrated by evaporation under reduced pressure. The resulting residue was washed three times with diethyl ether, and the powder thus formed was dried to obtain 1940 mg of the bis(trifluoromethanesulphonate) of the title compound as a powder.
Nuclear Magnetic Resonance Spectrum (hexadeuterated dimethyl sulphoxide + D₂O, 270 MHz) δ ppm:
1.81 (1H, doubled doublet of doublets, J = 13.2, 9.3 & 8.8 Hz);
2.82 (3H, singlet);
2.98 (1H, doublet of triplets, J = 13.2 & 7.8 Hz);
3.28 - 3.81 (11H, multiplet);
4.63 (1H, triplet, J = 8.8 Hz);
5.40 (2H, singlet);
7.72 (2H, doublet, J = 8.8 Hz);
8.28 (2H, doublet, J = 8.8 Hz).

The whole of this salt was dissolved in aqueous ethyl acetate, and an aqueous solution of sodium hydrogencarbonate was added to the resulting solution to make it alkaline. The ethyl acetate layer was separated, washed with an aqueous solution of sodium chloride, dried over anhydrous sodium sulphate and concentrated by evaporation under reduced pressure, to obtain 1.21 g of the title compound, in the form of an amorphous powder.
Infrared Absorption Spectrum (KBr), νₘₐₓ cm⁻¹:
   1648, 1605, 1544, 1520, 1440, 1346, 1304, 1273, 1232.
Nuclear Magnetic Resonance Spectrum (270MHz, CDCl₃ + D₂O) δ ppm:
   1.91 (1H, doubled doublet of doublets, J = 13.7,
   8.3 & 4.9 Hz);
   2.36 (3H, singlet);
   2.65 - 2.80 (2H, multiplet);
   3.08 (1H, doublet of doublets, J = 10.2 & 2.9 Hz);
   3.24 (1H, triplet, J = 8.3 Hz);
   3.32 - 3.44 (1H, multiplet);
   3.46 - 4.00 (8H, multiplet);
   5.21 (2H, singlet);
   7.56 (2H, doublet, J = 8.8 Hz);
   8.19 (2H, doublet, J = 8.8 Hz).

### PREPARATIONS 3 TO 21

The Compounds of Preparations 3 to 21 were synthesized in the same manner as described in Preparations 1 and 2.

### Preparation 3

(2S,4S)-4-Mercapto-1-(4-nitrobenzyloxycarbonyl)-2-[4-(4-nitrobenzyloxycarbonylamidino) homopiperazin-1-ylcarbonyl]pyrrolidine
Infrared Absorption Spectrum (KBr), νₘₐₓ cm⁻¹:
   1708, 1651, 1605, 1551, 1520, 1441, 1346, 1284.
Nuclear Magnetic Resonance Spectrum (270MHz, CDCl₃ + D₂O) δ ppm:
   1.75 - 1.98 (3H, multiplet);
   2.00 - 2.20 (1H, multiplet);
   2.66 - 2.82 (1H, multiplet);
   3.13 - 3.96 (8H, multiplet);
   4.06 - 4.15 (2H, multiplet);
   4.61 (1H, triplet, J = 8.0 Hz);
   5.10 - 5.25 (4H, multiplet);
   7.42 - 7.58 (4H, multiplet);
   8.16 - 8.23 (4H, multiplet).

### Preparation 4

(2S,4S)-4-Mercapto-1-methyl-2-[4-(4-nitrobenzyloxycarbonylamidino)homopiperazin-1-ylcarbonyl]pyrrolidine
Infrared Absorption Spectrum (KBr), νₘₐₓ cm⁻¹:
   1641, 1607, 1552, 1520, 1486, 1444, 1347, 1285.
Nuclear Magnetic Resonance Spectrum (270MHz, CDCl₃ + D₂O) δ ppm:
   1.81 - 2.01 (3H, multiplet);
   2.63 - 2.75 (1H, multiplet);
   2.80 - 2.88 (1H, multiplet);
   3.07 - 3.15 (1H, multiplet);
   3.23 - 3.93 (10H, multiplet);
   5.21 (2H, singlet);
   7.54 - 7.57 (2H, multiplet);
   8.17 - 8.22 (2H, multiplet).

### Preparation 5

(2S,4S)-4-Mercapto-1-(4-nitrobenzyloxycarbonyl)-2-[4-(4-nitrobenzyloxycarbonylguanidino)piperidin-1-ylcarbonyl]pyrrolidine

### Preparation 6

(2S,4S)-4-Mercapto-1-methyl-2-[4-(4-nitrobenzyloxycarbonylguanidino)piperidin-1-ylcarbonyl]pyrrolidine

### Preparation 7

(2S,4S)-4-Mercapto-1-(4-nitrobenzyloxycarbonyl)-2-[(3S)-3-(4-nitrobenzyloxycarbonylguanidino)pyrrolidin-1-ylcarbonyl]pyrrolidine

### Preparation 8

(2S,4S)-4-Mercapto-1-methyl-2-[(3S)-3-(4-nitrobenzyloxycarbonylguanidino)pyrrolidin-1-ylcarbonyl]pyrrolidine

### Preparation 9

(2S,4S)-4-Mercapto-1-(4-nitrobenzyloxycarbonyl)-2-[3-(4-nitrobenzyloxycarbonylguanidino)azetidin-1-ylcarbonyl]pyrrolidine

### Preparation 10

(2S,4S)-4-Mercapto-1-methyl-2-[3-(4-nitrobenzyloxycarbonylguanidino)azetidin-1-ylcarbonyl]pyrrolidine

### Preparation 13

(2S,4S)-4-Mercapto-1-(4-nitrobenzyloxycarbonyl)-2-[4-(methyl-4-nitrobenzyloxycarbonylamidino)piperazin-1-ylcarbonyl]pyrrolidine

### Preparation 14

(2S,4S)-4-Mercapto-1-methyl-2-[4-(methyl-4-nitrobenzyloxycarbonylamidino)piperazin-1-ylcarbonyl]pyrrolidine

### Preparation 15

(2S,4S)-4-Mercapto-1-(4-nitrobenzyloxycarbonyl)-2-[(3R)-3-(4-nitrobenzyloxycarbonylguanidino)pyrrolidin-1-yl-carbonyl]pyrrolidine

### Preparation 16

(2S,4S)-4-Mercapto-1-methyl-2-[(3R)-3-(4-nitrobenzyloxycarbonylguanidino)pyrrolidin-1-ylcarbonyl]pyrrolidine

### Preparation 17

(2S,4S)-4-Mercapto-1-(4-nitrobenzyloxycarbonyl)-2-[(3S)-4-(4-nitrobenzyloxycarbonylamidino)3-methylpiperazin-1-ylcarbonyl]pyrrolidine

### Preparation 18

(2S,4S)-4-Mercapto-1-methyl-2-[(3R)-4-(4-nitrobenzyloxycarbonylamidino)-3-methylpiperazin-1-ylcarbonyl]pyrrolidine

### PREPARATION 32

### 4-Nitrobenzyl (1R,5R,6S)-2-(diphenylphosphoryloxy)-6-[(1R)-1-hydroxyethyl]-1-methyl-1-carbapen-2-em-3-carboxylate

3.63 g of 4-nitrobenzyl (1R,5R,6S)-6-[(1R)-1-hydroxyethyl]-1-methyl-2-oxo-1-carbapenam-3-carboxylate were dissolved in 72 ml of dry acetonitrile, and then 2.28 ml of diphenylphosphoric acid chloride and 1.92 ml of diisopropylethylamine were added dropwise to the resulting solution, whilst stirring and ice-cooling. The resulting mixture was then stirred for a further 1 hour under the same conditions, after which it was concentrated by evaporation under reduced pressure. The resulting residue was dissolved in ethyl acetate and washed twice with water and then with an aqueous solution of sodium chloride, after which it was dried over anhydrous sodium sulphate and again concentrated by evaporation under reduced pressure. The residue was dissolved in 10 ml of ethyl acetate, and the resulting solution was left to stand to precipitate crystals. The mixture was diluted with 500 ml of diisopropyl ether, and crystals formed were collected by filtration and dried to obtain 5.34 g of the title compound, as colourless needle-like crystals, melting at 123 - 125°C.
Nuclear Magnetic Resonance Spectrum (270MHz, CDCl₃) δ ppm:
1.22 (3H, doublet, J = 7.2 Hz);
1.33 (3H, doublet, J = 6.6 Hz);
3.32 (1H, doublet of doublets, J = 6.6 & 2.6 Hz);
3.42 - 3.56 (1H, multiplet);
4.20 - 4.31 (2H, multiplet);
5.22 (1H, doublet, J = 13.8 Hz);
5.35 (1H, doublet, J = 13.8 Hz);
7.13 - 7.40 (10H, multiplet);
7.55 (1H, doublet, J = 8.6 Hz);
8.13 (2H, doublet, J = 8.6 Hz).

## Claims

1. Compounds of formula (I) and pharmaceutically acceptable salts and esters thereof: 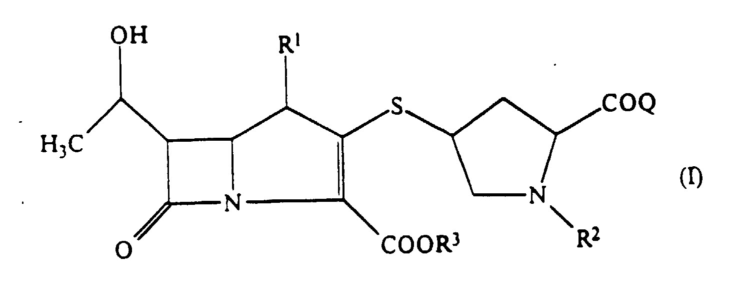 in which:
R¹ represents a hydrogen atom or a methyl group;
R² represents a hydrogen atom, an unsubstituted alkyl group having from 1 to 6 carbon atoms, a substituted alkyl group having from 1 to 6 carbon atoms which is substituted by at least one of substituents A, defined below, an alkenyl group having from 2 to 6 carbon atoms, an alkynyl group having from 2 to 6 carbon atoms, or a group of formula -C(=NH)R⁰,
where R⁰ represents a hydrogen atom or an alkyl group having from 1 to 6 carbon atoms;
R³ represents a hydrogen atom, a negative ion or a carboxy-protecting group; and
Q represents a group of formula (Q-VII)or (Q-VIII): 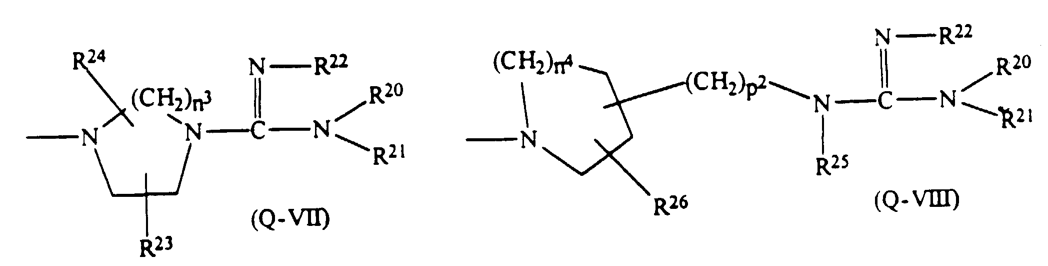 in which:
R²⁰, R²¹ and R²² are the same or different and each represents a hydrogen atom or an unsubstituted alkyl group having from 1 to 6 carbon atoms;
or
R²⁰ and R²¹ or R²⁰ and R²² together represent a group of formula - (CH₂)ₛ- (W)_{w,}- (CH₂)ₜ-, where s is 0, 1, 2 or 3, t is 0, 1, 2 or 3, W represents an oxygen or sulfur atom and w' is 0 or 1;
R²³ and R²⁴ are the same or different and each represents a hydrogen atom, a halogen atom, an unsubstituted alkyl group having from 1 to 6 carbon atoms, a substituted alkyl group having from 1 to 6 carbon atoms which is substituted by at least one of substituents C, defined below, a hydroxy group, a carboxy group, a carbamoyl group, an amino group, a cyano group or a carbamoyloxy group;
n³ is 1, 2 or 3;
R²⁵ represents a hydrogen atom, an unsubstituted alkyl group having from 1 to 6 carbon atoms, a substituted alkyl group having from 1 to 6 carbon atoms which is substituted by at least one of substituents C, defined below, an alkenyl group having from 2 to 6 carbon atoms, or an alkynyl group having from 2 to 6 carbon atoms;
R²⁶ represents a hydrogen atom, a halogen atom, an unsubstituted alkyl group having from 1 to 6 carbon atoms, a substituted alkyl group having from 1 to 6 carbon atoms which is substituted by at least one of substituents C, defined below, a hydroxy group, a carboxy group, a carbamoyl group, an amino group, a cyano group or a carbamoyloxy group;
n⁴ is 0, 1 or 2;
p² is 0 or 1; and
substituents A are selected from: hydroxy groups, carboxy groups, carbamoyl groups, carbamoyloxy groups, cyano groups, halogen atoms, oxygen atoms (to form an oxo group), alkoxy groups having from 1 to 6 carbon atoms, amino groups, alkylamino groups having from 1 to 6 carbon atoms and dialkylamino groups in which each alkyl part has from 1 to 6 carbon atoms; and
substituents C are selected from: hydroxy groups, carboxy groups, carbamoyl groups, cyano groups, halogen atoms, alkoxy groups having from 1 to 6 carbon atoms and amino groups.

2. A compound according to Claim 1, in which:
R¹ represents a hydrogen atom or a methyl group;
R² represents a hydrogen atom, an unsubstituted alkyl group having from 1 to 3 carbon atoms, a substituted alkyl group which has from 1 to 3 carbon atoms and which is substituted by at least one of substituents A¹, defined below, an alkenyl group having 3 or 4 carbon atoms, an alkynyl group having 3 or 4 carbon atoms, or a group of formula -C(=NH)R⁰,
where R⁰ represents a hydrogen atom or an alkyl group having from 1 to 3 carbon atoms;
R³ represents a hydrogen atom or a negative ion; and
Q represents a group of formula (Q-VII) or (Q-VIII), as defined in Claim 1, in which:
R²⁰, R²¹ and R²² are the same or different and each represents a hydrogen atom or an unsubstituted alkyl group having from 1 to 3 carbon atoms;
or
R²⁰ and R²¹ or R²⁰ and R²² together represent a group of formula -(CH₂)ₛ- (W)_{w},- (CH₂)ₜ-, where s is 1 or 2, t is 1 or 2, W represents an oxygen or sulfur atom and w' is 0 or 1, provided that (s + w' + t) is 2, 3 or 4;
R²³ and R²⁴ are the same or different and each represents a hydrogen atom, a halogen atom, an unsubstituted alkyl group having from 1 to 3 carbon atoms, a substituted alkyl group which has from 1 to 3 carbon atoms and which is substituted by at least one of substituents C¹ defined below, a hydroxy group, a carboxy group, a carbamoyl group, an amino group, a cyano group or a carbamoyloxy group;
n³ is 1, 2 or 3;
R²⁵ represents a hydrogen atom, an unsubstituted alkyl group having from 1 to 3 carbon atoms, a substituted alkyl group which has from 1 to 3 carbon atoms and which is substituted by at least one of substituents C¹, defined below, an alkenyl group having 3 or 4 carbon atoms, or an alkynyl group having 3 or 4 carbon atoms;
R²⁶ represents a hydrogen atom, a halogen atom, an unsubstituted alkyl group having from 1 to 3 carbon atoms, a substituted alkyl group which has from 1 to 3 carbon atoms and which is substituted by at least one of substituents C¹, defined below, a hydroxy group, a carboxy group, a carbamoyl group, an amino group, a cyano group or a carbamoyloxy group;
n⁴ is 0, 1 or 2;
p² is 0 or 1; and
substituents A¹ are selected from hydroxy groups, carboxy groups, carbamoyl groups, carbamoyloxy groups, cyano groups, halogen atoms and amino groups; and
substituents C¹ are selected from hydroxy groups, carboxy groups, carbamoyl groups, halogen atoms and amino groups.

3. A compound according to Claim 1, in which:
R¹ represents a hydrogen atom or a methyl group;
R² represents a hydrogen atom, an unsubstituted alkyl group having from 1 to 3 carbon atoms, a substituted alkyl group which has from 1 to 3 carbon atoms and which is substituted by at least one of substituents A², defined below, or a group of formula -C(=NH)R⁰,
where R⁰ represents a hydrogen atom or an alkyl group having from 1 to 3 carbon atoms;
R³ represents a hydrogen atom or a negative ion; and
Q represents a group of formula (Q-VII) or (Q-VIII), as defined in Claim 1, in which:
R²⁰, R²¹ and R²² are the same or different and each represents a hydrogen atom or an unsubstituted alkyl group having from 1 to 3 carbon atoms;
or
R²⁰ and R²¹ or R²⁰ and R²² together represent a group of formula -(CH₂)ₛ-(W)_{w},-(CH₂)ₜ-, where s is 1 or 2, t is 1 or 2, W represents an oxygen or sulfur atom and w' is 0 or 1, provided that (s + w' + t) is 2, 3 or 4;
R²³ and R²⁴ are the same or different and each represents a hydrogen atom, a halogen atom, a hydroxy group, a carbamoyl group, a carboxy group, an unsubstituted alkyl group having from 1 to 3 carbon atoms or a substituted alkyl group which has from 1 to 3 carbon atoms and which is substituted by at least one substituent selected from hydroxy groups, amino groups and carbamoyl groups;
n³ is 1, 2 or 3;
R²⁵ represents a hydrogen atom or a methyl group;
R²⁶ represents a hydrogen atom;
n⁴ is 0, 1 or 2;
p² is 0 or 1; and
substituents A² are selected from hydroxy groups, carboxy groups, carbamoyl groups, carbamoyloxy groups, halogen atoms and amino groups.

4. A compound according to Claim 1, in which Q represents a group of formula (Q-VII).

5. A compound according to Claim 1, in which R¹ represents a methyl group.

6. A compound according to Claim 1, in which Q represents a group of formula (Q-VII), and R¹ represents a methyl group.

7. A compound according to Claim 2, in which Q represents a group of formula (Q-VII), and R¹ represents a methyl group.

8. A compound according to Claim 3, in which Q represents a group of formula (Q-VII), and R¹ represents a methyl group.

9. 2-[2-(4-Amidinopiperazin-1-ylcarbonyl)pyrrolidin-4-ylthio]-6-(1-hydroxyethyl)-1-methyl-1-carbapen-2-em-3-carboxylic acid and pharmaceutically acceptable salts thereof.

10. 2-[2-(4-Amidinopiperazin-1-ylcarbonyl)-1-methylpyrrolidin-4-ylthio]-6-(1-hydroxyethyl)-1-methyl-1-carbapen-2-em-3-carboxylic acid and pharmaceutically acceptable salts thereof.

11. 2-[2-(4-Amidinohomopiperazin-1-ylcarbonyl)pyrrolidin-4-ylthio]-6-(1-hydroxyethyl)-1-methyl-1-carbapen-2-em-3-carboxylic acid and pharmaceutically acceptable salts thereof.

12. 2-[2-(4-Amidinohomopiperazin-1-ylcarbonyl)-1-methylpyrrolidin-4-ylthio]-6-(1-hydroxyethyl)-1-methyl-1-carbapen-2-em-3-carboxylic acid and pharmaceutically acceptable salts thereof.

13. A pharmaceutical composition comprising a pharmaceutically acceptable carrier, diluent or adjuvant in admixture with an effective amount of an antibiotic, in which the antibiotic is at least one compound of formula (I) or a pharmaceutically acceptable salt or ester thereof, as claimed in any one of Claims 1 to 12.

14. A compound of formula (I) or a pharmaceutically acceptable salt or ester thereof, as claimed in any one of Claims 1 to 12, for use as a pharmaceutical.

15. The use of a compound of formula (I) or a pharmaceutically acceptable salt or ester thereof, as claimed in any one of Claims 1 to 12, for the manufacture of a medicament for the treatment or prevention of microbial infections.

16. A process for preparing a compound of formula (I) or a pharmaceutically acceptable salt or ester thereof, as claimed in any one of Claims 1 to 12, which comprises the steps:
reacting a carbapenem compound of formula (II) : 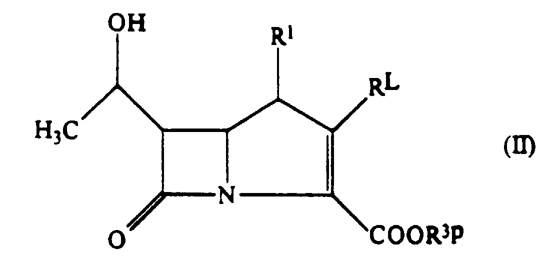 (in which R¹ is as defined in Claim 1, R^{L} represents a sulfonyloxy or phosphoryloxy group or a group of formula -S(O)R^{L1}, R^{L1} represents an alkyl group, a haloalkyl group, an alkanoylaminoalkyl group, an alkenoylaminoalkyl group, an aryl group or an aromatic heterocyclic group, and R^{3p} represents a carboxy-protecting group) with a pyrrolidine derivative of formula (III): 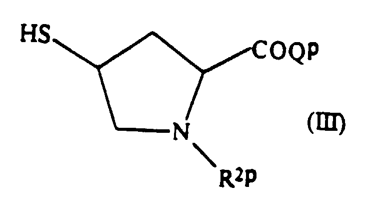 (in which R^{2p} represents any of the groups or atoms represented by R² or any such group or atom which has been protected, and Q^{p} represents any of the groups or atoms represented by Q or any such group or atom which has been protected and, where the group Q^{p} contains a quaternary nitrogen atom, the compound is accompanied by a balancing anion),
and, if necessary, removing any protecting groups,
and optionally salifying and/or esterifying the product.

## Patentansprüche

1. Verbindungen der Formel (I) und pharmazeutisch geeignete Salze und Ester davon: 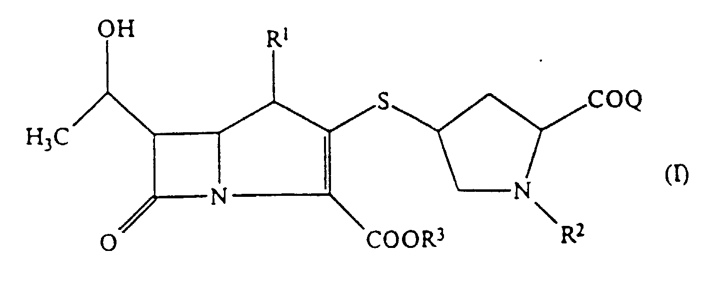 worin:
R¹ ein Wasserstoffatom oder eine Methylgruppe bedeutet,
R² ein Wasserstoffatom, eine unsubstituierte Alkylgruppe mit 1 bis 6 Kohlenstoffatomen, eine substituierte Alkylgruppe mit 1 bis 6 Kohlenstoffatomen, die mit mindestens einem der nachstehend definierten Substituenten substituiert ist, eine Alkenylgruppe mit 2 bis 6 Kohlenstoffatomen, eine Alkinylgruppe mit 2 bis 6 Kohlenstoffatomen oder eine Gruppe der Formel -C(=NH)R⁰ bedeutet,
wobei R⁰ ein Wasserstoffatom oder eine Alkylgruppe mit 1 bis 6 Kohlenstoffatomen darstellt;
R³ ein Wasserstoffatom, ein negatives Ion oder eine Carboxy-Schutzgruppe bedeutet, und
Q eine Gruppe der Formel (Q-VII) oder (Q-VIII) darstellt: 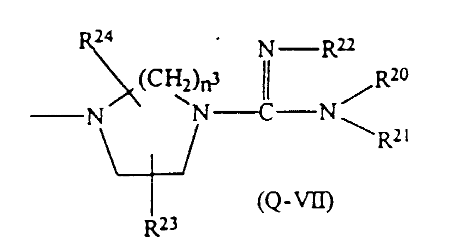 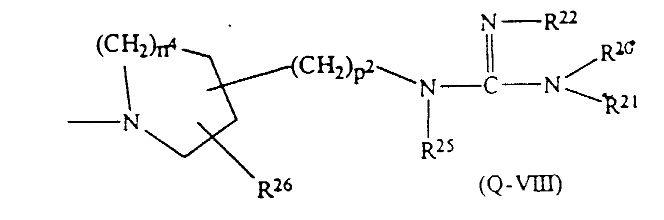 worin
R²⁰, R²¹ und R²² gleich oder verschieden sind und jeweils ein Wasserstoffatom oder eine unsubstituierte Alkylgruppe mit 1 bis 6 Kohlenstoffatomen bedeuten,
oder
R²⁰ und R²¹ oder R²⁰ und R²² zusammen eine Gruppe der Formel -(CH₂)ₛ-(W)_{w},-(CH₂)ₜ- darstellen, wobei s 0, 1, 2 oder 3 ist, t 0, 1, 2 oder 3 ist, W ein Sauerstoff- oder Schwefelatom bedeutet und w' 0 oder 1 ist,
R²³ und R²⁴ gleich oder verschieden sind und jeweils ein Wasserstoffatom, ein Halogenatom, eine unsubstituierte Alkylgruppe mit 1 bis 6 Kohlenstoffatomen, eine substituierte Alkylgruppe mit 1 bis 6 Kohlenstoffatomen, die mit mindestens einem der nachstehend definierten Substituenten C substituiert ist, eine Hydroxygruppe, eine Carboxygruppe, eine Carbamoylgruppe, eine Aminogruppe, eine Cyangruppe oder eine Carbamoyloxygruppe bedeuten,
n³ 1, 2 oder 3 ist,
R²⁵ ein Wasserstoffatom, eine unsubstituierte Alkylgruppe mit 1 bis 6 Kohlenstoffatomen, eine substituierte Alkylgruppe mit 1 bis 6 Kohlenstoffatomen, die mit mindestens einem der nachstehend definierten Substituenten C substituiert ist, eine Alkenylgruppe mit 2 bis 6 Kohlenstoffatomen oder eine Alkinylgruppe mit 2 bis 6 Kohlenstoffatomen bedeutet,
R²⁶ ein Wasserstoffatom, ein Halogenatom, eine unsubstituierte Alkylgruppe mit 1 bis 6 Kohlenstoffatomen, eine substituierte Alkylgruppe mit 1 bis 6 Kohlenstoffatomen, die mit mindestens einem der nachstehend definierten Substituenten C substituiert ist, eine Hydroxygruppe, eine Carboxygruppe, eine Carbamoylgruppe, eine Aminogruppe, eine Cyangruppe oder eine Carbamoyloxygruppe bedeutet,
n⁴ 0, 1 oder 2 ist,
p² 0 oder 1 ist, und
die Substituenten A unter Hydroxygruppen, Carboxygruppen, Carbamoylgruppen, Carbamoyloxygruppen, Cyangruppen, Halogenatomen, Sauerstoffatomen (unter Bildung einer Oxogruppe), Alkoxygruppen mit 1 bis 6 Kohlenstoffatomen, Aminogruppen, Alkylaminogruppen mit 1 bis 6 Kohlenstoffatomen, und Dialkylaminogruppen, in denen jeder Alkylteil 1 bis 6 Kohlenstoffatome aufweist, ausgewählt sind und
die Substituenten C unter Hydroxygruppen, Carboxygruppen, Carbamoylgruppen, Cyangruppen, Halogenatomen, Alkoxygruppen mit 1 bis 6 Kohlenstoffatomen und Aminogruppen ausgewählt sind.

2. Verbindung nach Anspruch 1, worin
R¹ ein Wasserstoffatom oder eine Methylgruppe bedeutet,
R² ein Wasserstoffatom, eine unsubstituierte Alkylgruppe mit 1 bis 3 Kohlenstoffatomen, eine substituierte Alkylgruppe mit 1 bis 3 Kohlenstoffatomen, die mit mindestens einem der nachstehend definierten Substituenten A¹ substituiert ist, eine Alkenylgruppe mit 3 oder 4 Kohlenstoffatomen, eine Alkinylgruppe mit 3 oder 4 Kohlenstoffatomen oder eine Gruppe der Formel -C(=NH)R⁰ bedeutet,
wobei R⁰ ein Wasserstoffatom oder eine Alkylgruppe mit 1 bis 3 Kohlenstoffatomen darstellt,
R³ ein Wasserstoffatom oder ein negatives Ion bedeutet, und
Q eine Gruppe der Formel (Q-VII) oder (Q-VIII) der in Anspruch 1 gegebenen Definition darstellt, wobei
R²⁰, R²¹ und R²² gleich oder verschieden sind und jeweils ein Wasserstoffatom oder eine unsubstituierte Alkylgruppe mit 1 bis 3 Kohlenstoffatomen bedeuten,
oder
R²⁰ und R²¹ oder R²⁰ und R²² zusammen eine Gruppe der Formel -(CH₂)ₛ-(W)_{w},-(CH₂)ₜ- darstellen, wobei s 1 oder 2 ist, t 1 oder 2 ist, W ein Sauerstoff- oder Schwefelatom bedeutet und w' 0 oder 1 ist, mit der Maßgabe, daß (s + w' + t) 2, 3 oder 4 ist,
R²³ und R²⁴ gleich oder verschieden sind und jeweils ein Wasserstoffatom, ein Halogenatom, eine unsubstituierte Alkylgruppe mit 1 bis 3 Kohlenstoffatomen, eine substituierte Alkylgruppe mit 1 bis 3 Kohlenstoffatomen, die mit mindestens einem der nachstehend definierten Substituenten C¹ substituiert ist, eine Hydroxygruppe, eine Carboxygruppe, eine Carbamoylgruppe, eine Aminogruppe, eine Cyangruppe oder eine Carbamoyloxygruppe bedeuten,
n³ 1, 2 oder 3 bedeutet,
R²⁵ ein Wasserstoffatom, eine unsubstituierte Alkylgruppe mit 1 bis 3 Kohlenstoffatomen, eine substituierte Alkylgruppe mit 1 bis 3 Kohlenstoffatomen, die mit mindestens einem der nachstehend definierten Substituenten C¹ substituiert ist, eine Alkenylgruppe mit 3 oder 4 Kohlenstoffatomen oder eine Alkinylgruppe mit 3 oder 4 Kohlenstoffatomen bedeutet,
R²⁶ ein Wasserstoffatom, ein Halogenatom, eine unsubstituierte Alkylgruppe mit 1 bis 3 Kohlenstoffatomen, eine substituierte Alkylgruppe mit 1 bis 3 Kohlenstoffatomen, die mit mindestens einem der nachstehend definierten Substituenten C¹ substituiert ist, eine Hydroxygruppe, eine Carboxygruppe, eine Carbamoylgruppe, eine Aminogruppe, eine Cyangruppe oder eine Carbamoyloxygruppe bedeutet,
n⁴ 0, 1 oder 2 ist,
p² 0 oder 1 ist, und
die Substituenten A¹ unter Hydroxygruppen, Carboxygruppen, Carbamoylgruppen, Carbamoyloxygruppen, Cyangruppen, Halogenatomen und Aminogruppen ausgewählt sind und
die Substituenten C¹ unter Hydroxygruppen, Carboxygruppen, Carbamoylgruppen, Halogenatomen und Aminogruppen ausgewählt sind.

3. Verbindung nach Anspruch 1, worin
R¹ ein Wasserstoffatom oder eine Methylgruppe bedeutet,
R² ein Wasserstoffatom, eine unsubstituierte Alkylgruppe mit 1 bis 3 Kohlenstoffatomen, eine substituierte Alkylgruppe mit 1 bis 3 Kohlenstoffatomen, die mit mindestens einem der nachstehend definierten Substituenten A² substituiert ist oder eine Gruppe der Formel -C(=NH)R⁰ bedeutet,
wobei R⁰ ein Wasserstoffatom oder eine Alkylgruppe mit 1 bis 3 Kohlenstoffatomen darstellt,
R³ ein Wasserstoffatom oder ein negatives Ion bedeutet und
Q eine Gruppe der Formel (Q-VII) oder (Q-VIII) gemäß der Definition in Anspruch 1 bedeutet, worin
R²⁰, R²¹ und R²² gleich oder verschieden sind und jeweils ein Wasserstoffatom oder eine unsubstituierte Alkylgruppe mit 1 bis 3 Kohlenstoffatomen bedeuten,
oder
R²⁰ und R²¹ oder R²⁰ und R²² zusammen eine Gruppe der Formel -(CH₂)ₛ-(W)_{w},-(CH₂)ₜ- darstellen, wobei s 1 oder 2 ist, t 1 oder 2 ist, W ein Sauerstoff- oder Schwefelatom bedeutet und w' 0 oder 1 ist, mit der Maßgabe, daß (s + w' + t) 2, 3 oder 4 bedeutet,
R²³ und R²⁴ gleich oder verschieden sind und jeweils ein Wasserstoffatom, ein Halogenatom, eine Hydroxygruppe eine Carbamoylgruppe, eine carboxygruppe, eine unsubstituierte Alkylgruppe mit 1 bis 3 Kohlenstoffatomen oder eine substituierte Alkylgruppe, die 1 bis 3 Kohlenstoffatome aufweist und die mit mindestens einem unter Hydroxygruppen, Aminogruppen und Carbamoylgruppen ausgewählten Substituenten substituiert ist, bedeuten,
n³ 1, 2 oder 3 ist,
R²⁵ ein Wasserstoffatom oder eine Methylgruppe bedeutet,
R²⁶ ein Wasserstoffatom bedeutet,
n⁴ 0, 1 oder 2 ist,
p² 0 oder 1 ist, und
die Substituenten A² unter Hydroxygruppen, Carboxygruppen, Carbamoylgruppen, Carbamoyloxygruppen, Halogenatomen und Aminogruppen ausgewählt sind.

4. Verbindung nach Anspruch 1, in der Q eine Gruppe der Formel (Q-VII) bedeutet.

5. Verbindung nach Anspruch 1, in der R¹ eine Methylgruppe bedeutet.

6. Verbindung nach Anspruch 1, in der Q eine Gruppe der Formel (Q-VII) bedeutet und R¹ eine Methylgruppe bedeutet.

7. Verbindung nach Anspruch 2, in der Q eine Gruppe der Formel (Q-VII) bedeutet und R¹ eine Methylgruppe bedeutet.

8. Verbindung nach Anspruch 3, in der Q eine Gruppe der Formel (Q-VII) bedeutet und R¹ eine Methylgruppe bedeutet.

9. 2-[2-(4-Amidinopiperazin-1-ylcarbonyl)pyrrolidin-4-ylthio]-6-(1-hydroxyethyl)-1-methyl-1-carbapen-2-em-3-carbonsäure und deren pharmazeutisch geeignete Salze.

10. 2-[2-(4-Amidinopiperazin-1-ylcarbonyl)-1-methylpyrrolidin-4-ylthio]-6-(1-hydroxyethyl)-1-methyl-1-carbapen-2-em-3-carbonsäure und deren pharmazeutisch geeignete Salze.

11. 2-[2-(4-Amidinohomopiperazin-1-ylcarbonyl)pyrrolidin-4-ylthio]-6-(1-hydroxyethyl)-1-methyl-1-carbapen-2-em-3-carbonsäure und deren pharmazeutisch geeignete Salze.

12. 2-[2-(4-Amidinohomopiperazin-1-ylcarbonyl)-1-methylpyrrolidin-4-ylthio]-6-(1-hydroxyethyl)-1-methyl-1-carbapen-2-em-3-carbonsäure und deren pharmazeutisch geeignete Salze.

13. Arzneimittelzusammensetzung, die ein pharmazeutisch geeignetes Trägermaterial, Verdünnungsmittel oder Adjuvans im Gemisch mit einer wirksamen Menge eines Antibiotikums umfaßt, wobei das Antibiotikum mindestens eine Verbindung der Formel (I) oder ein pharmazeutisch geeignetes Salz oder ein pharmazeutisch geeigneter Ester dieser Verbindung gemäß einem der Ansprüche 1 bis 12 ist.

14. Verbindung der Formel (I) oder pharmazeutisch geeignetes Salz oder pharmazeutisch geeigneter Ester dieser Verbindung gemäß einem der Ansprüche 1 bis 12 zur Verwendung als Arzneimittel.

15. Verwendung einer Verbindung der Formel (I) oder eines pharmazeutisch geeigneten Salzes oder Esters dieser Verbindung gemäß einem der Ansprüche 1 bis 12 zur Herstellung eines Arzneimittels zur Behandlung oder Verhütung von mikrobiellen Infektionen.

16. Verfahren zur Herstellung einer Verbindung der Formel (I) oder eines pharmazeutisch geeigneten Salzes oder Esters dieser Verbindung gemäß einem der Ansprüche 1 bis 12, welches folgende Stufen umfaßt:
Umsetzen einer Carbapenemverbindung der Formel (II): 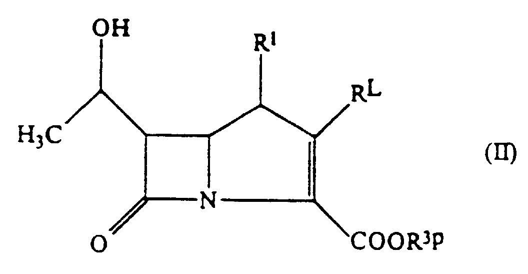 (in der R¹ wie in Anspruch 1 definiert ist, R^{L} eine Sulfonyloxy- oder Phosphoryloxygruppe oder eine Gruppe der Formel -S(O)R^{L1} bedeutet, R^{L1} eine Alkylgruppe, eine Halogenalkylgruppe, eine Alkanoylaminoalkylgruppe, eine Alkenoylaminoalkylgruppe, eine Arylgruppe oder eine aromatische heterocyclische Gruppe bedeutet und R^{3p} eine Carboxyschutzgruppe darstellt) mit einem Pyrrolidinderivat der Formel (III): 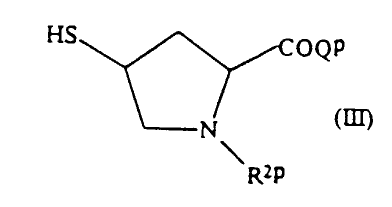 (in der R^{2p} irgendeine der durch R² dargestellten Gruppen oder Atome oder irgendeine solche Gruppe oder ein solches Atom bedeutet, die bzw. das geschützt ist, und Q^{p} irgendeine der durch Q dargestellten Gruppen oder Atome oder irgendeine solche Gruppe oder irgendein solches Atom, die bzw. das geschützt ist, bedeutet, und, wobei dann, wenn die Gruppe Q^{p} ein quaternäres Stickstoffatom enthält, die Verbindung mit einem ladungsausgleichenden Anion versehen ist,
und erforderlichenfalls Entfernung jeglicher Schutzgruppen,
und gegebenenfalls Überführen des Produkts in ein Salz und/oder Verestern des Produkts.

## Revendications

1. Composés de formule (I) et sels et esters pharmaceutiquement acceptables de ceux-ci : 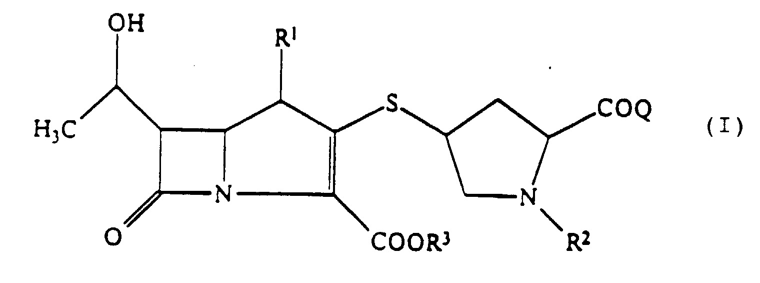 dans laquelle :
R¹ représente un atome d'hydrogène ou un groupe méthyle;
R² représente un atome d'hydrogène, un groupe alkyle non substitué ayant de 1 à 6 atomes de carbone, un groupe alkyle substitué ayant de 1 à 6 atomes de carbone qui est substitué par au moins un des substituants A, définis ci-dessous, un groupe alcényle ayant de 2 à 6 atomes de carbone, un groupe alcynyle ayant de 2 à 6 atomes de carbone, ou un groupe de formule -C(=NH)R⁰,
R⁰ représentant un atome d'hydrogène ou un groupe alkyle ayant de 1 à 6 atomes de carbone;
R³ représente un atome d'hydrogène, un ion négatif ou un groupe protecteur de carboxy; et
Q représente un groupe de formule (Q-VII) ou (Q-VIII): 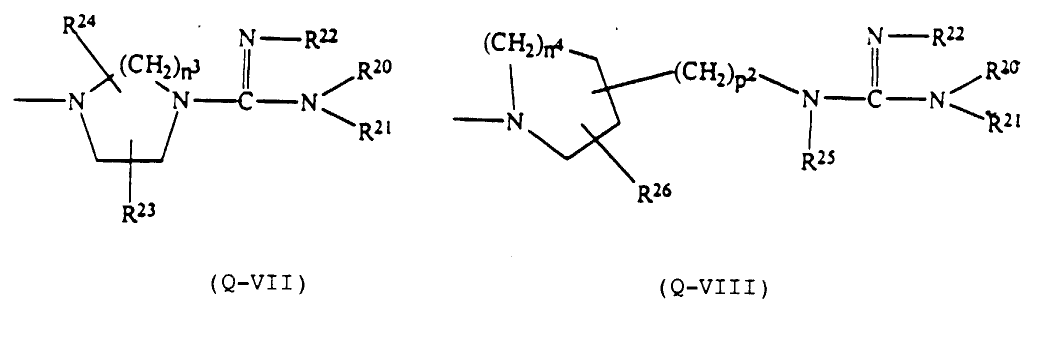 où :
R²⁰, R²¹ et R²² sont identiques ou différents et représentent chacun un atome d'hydrogène ou un groupe alkyle non substitué ayant de 1 à 6 atomes de carbone;
ou
R²⁰ et R²¹ ou bien R²⁰ et R²² ensemble représentent un groupe de formule -(CH₂)ₛ-(W)_{w},-(CH₂)ₜ-, où s a une valeur égale à 0, 1, 2 ou 3, t a une valeur égale à 0, 1, 2 ou 3, W représente un atome d'oxygène ou de soufre et w' a une valeur égale à 0 ou 1;
R²³ et R²⁴ sont identiques ou différents et représentent chacun un atome d'hydrogène, un atome d'halogène, un groupe alkyle non substitué ayant de 1 à 6 atomes de carbone, un groupe alkyle substitué ayant de 1 à 6 atomes de carbone qui est substitué par au moins un des substituants C, définis ci-dessous, un groupe hydroxy, un groupe carboxy, un groupe carbamoyle, un groupe amino, un groupe cyano ou un groupe carbamoyloxy;
n³ a une valeur égale à 1, 2 ou 3;
R²⁵ représente un atome d'hydrogène, un groupe alkyle non substitué ayant de 1 à 6 atomes de carbone, un groupe alkyle substitué ayant de 1 à 6 atomes de carbone qui est substitué par au moins un des substituants C, définis ci-dessous, un groupe alcényle ayant de 2 à 6 atomes de carbone, ou un groupe alcynyle ayant de 2 à 6 atomes de carbone;
R²⁶ représente un atome d'hydrogène, un atome d'halogène, un groupe alkyle non substitué ayant de 1 à 6 atomes de carbone, un groupe alkyle substitué ayant de 1 à 6 atomes de carbone qui est substitué par au moins un des substituants C, définis ci-dessous, un groupe hydroxy, un groupe carboxy, un groupe carbamoyle, un groupe amino, un groupe cyano ou un groupe carbamoyloxy;
n⁴ a une valeur égale à 0, 1 ou 2;
p² a une valeur égale à 0 ou 1; et
les substituants A sont choisis parmi : les groupes hydroxy, les groupes carboxy, les groupes carbamoyles, les groupes carbamoyloxy, les groupes cyano, les atomes d'halogène, les atomes d'oxygène (pour former un groupe oxo), les groupes alcoxy ayant de 1 à 6 atomes de carbone, les groupes amino, les groupes alkylamino ayant de 1 à 6 atomes de carbone et les groupes dialkylamino dans lesquels chaque fragment alkyle possède 1 à 6 atomes de carbone; et
les substituants C sont choisis parmi : les groupes hydroxy, les groupes carboxy, les groupes carbamoyles, les groupes cyano, les atomes d'halogène, les groupes alcoxy ayant de 1 à 6 atomes de carbone et les groupes amino.

2. Composé selon la revendication 1, dans lequel :
R¹ représente un atome d'hydrogène ou un groupe méthyle;
R² représente un atome d'hydrogène, un groupe alkyle non substitué ayant de 1 à 3 atomes de carbone, un groupe alkyle substitué qui a de 1 à 3 atomes de carbone et qui est substitué par au moins un des substituants A¹, définis ci-dessous, un groupe alcényle ayant 3 ou 4 atomes de carbone, un groupe alcynyle ayant 3 ou 4 atomes de carbone, ou un groupe de formule -C(=NH)R⁰,
R⁰ représentant un atome d'hydrogène ou un groupe alkyle ayant de 1 à 3 atomes de carbone;
R³ représente un atome d'hydrogène ou un ion négatif; et
Q représente un groupe de formule (Q-VII) ou (Q-VIII), tel que défini dans la revendication 1, où :
R²⁰, R²¹ et R²² sont identiques ou différents et représentent chacun un atome d'hydrogène ou un groupe alkyle non substitué ayant de 1 à 3 atomes de carbone;
ou
R²⁰ et R²¹ ou bien R²⁰ et R²² ensemble représentent un groupe de formule -(CH₂)ₛ-(W)_{w},-(CH₂)ₜ-, où s vaut 1 ou 2, t vaut 1 ou 2, W représente un atome d'oxygène ou de soufre et w' vaut 0 ou 1, à condition que la somme (s + w' + t) soit égale à 2, 3 ou 4;
R²³ et R²⁴ sont identiques ou différents et représentent chacun un atome d'hydrogène, un atome d'halogène, un groupe alkyle non substitué ayant de 1 à 3 atomes de carbone, un groupe alkyle substitué qui a de 1 à 3 atomes de carbone et qui est substitué par au moins un des substituants C¹ définis ci-dessous, un groupe hydroxy, un groupe carboxy, un groupe carbamoyle, un groupe amino, un groupe cyano ou un groupe carbamoyloxy;
n³ vaut 1, 2 ou 3;
R²⁵ représente un atome d'hydrogène, un groupe alkyle non substitué ayant de 1 à 3 atomes de carbone, un groupe alkyle substitué qui a de 1 à 3 atomes de carbone et qui est substitué par au moins un des substituants C¹, définis ci-dessous, un groupe alcényle ayant 3 ou 4 atomes de carbone, ou un groupe alcynyle ayant 3 ou 4 atomes de carbone;
R²⁶ représente un atome d'hydrogène, un atome d'halogène, un groupe alkyle non substitué ayant de 1 à 3 atomes de carbone, un groupe alkyle substitué qui a de 1 à 3 atomes de carbone et qui est substitué par au moins un des substituants C¹, définis ci-dessous, un groupe hydroxy, un groupe carboxy, un groupe carbamoyle, un groupe amino, un groupe cyano ou un groupe carbamoyloxy;
n⁴ vaut 0, 1 ou 2;
p² vaut 0 ou 1; et
les substituants A¹ sont choisis parmi les groupes hydroxy, les groupes carboxy, les groupes carbamoyles, les groupes carbamoyloxy, les groupes cyano, les atomes d'halogène et les groupes amino; et
les substituants C¹ sont choisis parmi les groupes hydroxy, les groupes carboxy, les groupes carbamoyles, les atomes d'halogène et les groupes amino.

3. Composé selon la revendication 1, dans lequel :
R¹ représente un atome d'hydrogène ou un groupe méthyle;
R² représente un atome d'hydrogène, un groupe alkyle non substitué ayant de 1 à 3 atomes de carbone, un groupe alkyle substitué qui a de 1 à 3 atomes de carbone et qui est substitué par au moins un des substituants A², définis ci-dessous, ou un groupe de formule -C(=NH)R⁰,
R⁰ représentant un atome d'hydrogène ou un groupe alkyle ayant de 1 à 3 atomes de carbone;
R³ représente un atome d'hydrogène ou un ion négatif; et
Q représente un groupe de formule (Q-VII) ou (Q-VIII), tel que défini dans la revendication 1, où :
R²⁰, R²¹ et R²² sont identiques ou différents et représentent chacun un atome d'hydrogène ou un groupe alkyle non substitué ayant de 1 à 3 atomes de carbone;
ou
R²⁰ et R²¹ ou bien R²⁰ et R²² ensemble représentent un groupe de formule -(CH₂)ₛ-(W)_{w},-(CH₂)ₜ-, où s vaut 1 ou 2, t vaut 1 ou 2, W représente un atome d'oxygène ou de soufre et w' vaut 0 ou 1, à condition que la somme (s + w' + t) soit égale à 2, 3 ou 4;
R²³ et R²⁴ sont identiques ou différents et représentent chacun un atome d'hydrogène, un atome d'halogène, un groupe hydroxy, un groupe carbamoyle, un groupe carboxy, un groupe alkyle non substitué ayant de 1 à 3 atomes de carbone ou un groupe alkyle substitué qui a de 1 à 3 atomes de carbone et qui est substitué par au moins un substituant choisi parmi les groupes hydroxy, les groupes amino et les groupes carbamoyles;
n³ vaut 1, 2 ou 3;
R²⁵ représente un atome d'hydrogène ou un groupe méthyle;
R²⁶ représente un atome d'hydrogène;
n⁴ vaut 0, 1 ou 2;
p² vaut O ou 1; et
les substituants A² sont choisis parmi les groupes hydroxy, les groupes carboxy, les groupes carbamoyles, les groupes carbamoyloxy, les atomes d'halogène et les groupes amino.

4. Composé selon la revendication 1, dans lequel Q représente un groupe de formule (Q-VII).

5. Composé selon la revendication 1, dans lequel R¹ représente un groupe méthyle.

6. Composé selon la revendication 1, dans lequel Q représente un groupe de formule (Q-VII), et R¹ représente un groupe méthyle.

7. Composé selon la revendication 2, dans lequel Q représente un groupe de formule (Q-VII), et R¹ représente un groupe méthyle.

8. Composé selon la revendication 3, dans lequel Q représente un groupe de formule (Q-VII), et R¹ représente un groupe méthyle.

9. Acide 2-[2-(4-amidinopipérazin-1-ylcarbonyl)pyrrolidin-4-ylthio]-6-(1-hydroxyéthyl)-1-méthyl-1-carbapen-2-em-3-carboxylique et sels pharmaceutiquement acceptables de celui-ci.

10. Acide 2-[2-(4-amidinopipérazin-1-ylcarbonyl)-1méthylpyrrolidin-4-ylthio]-6-(1-hydroxyéthyl)-1-méthyl-1-carbapen-2-em-3-carboxylique et sels pharmaceutiquement acceptables de celui-ci.

11. Acide 2-[2-(4-amidinohomopipérazin-1-ylcarbonyl)pyrrolidin-4-ylthio]-6-(1-hydroxyéthyl)-1-méthyl-1-carbapen-2-em-3-carboxylique et sels pharmaceutiquement acceptables de celui-ci.

12. Acide 2-[2-(4-amidinohomopipérazin-1-ylcarbonyl)-1-méthylpyrrolidin-4-ylthio]-6-(1-hydroxyéthyl)-1-méthyl-1-carbapen-2-em-3-carboxylique et sels pharmaceutiquement acceptables de celui-ci.

13. Composition pharmaceutique comprenant un support, diluant, ou adjuvant, pharmaceutiquement acceptable, en mélange avec une quantité efficace d'un antibiotique, dans laquelle l'antibiotique est au moins un composé de formule (I) ou un sel ou ester pharmaceutiquement acceptable de celui-ci, tel que revendiqué dans l'une quelconque des revendications 1 à 12.

14. Composé de formule (I) ou sel ou ester pharmaceutiquement acceptable de celui-ci, tel que revendiqué dans l'une quelconque des revendications 1 à 12, destiné à être utilisé comme médicament.

15. Utilisation d'un composé de formule (I) ou d'un sel ou ester pharmaceutiquement acceptable de celui-ci, tel que revendiqué dans l'une quelconque des revendications 1 à 12, pour la préparation d'un médicament destiné au traitement ou à la prévention d'infections microbiennes.

16. Procédé de préparation d'un composé de formule (I) ou d'un sel ou ester pharmaceutiquement acceptable de celui-ci, tel que revendiqué dans l'une quelconque des revendications 1 à 12, qui comporte les étapes consistant :
à faire réagir un dérivé de carbapenem de formule (II) : 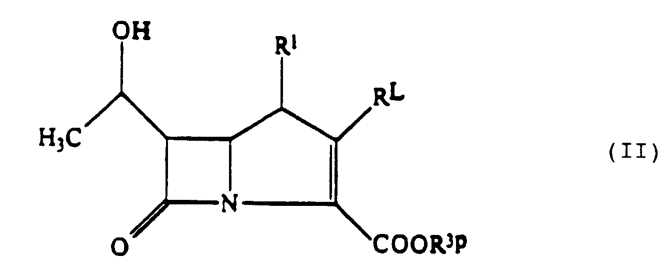 (dans laquelle R¹ est tel que défini dans la revendication 1, R^{L} représente un groupe sulfonyloxy ou phosphoryloxy ou un groupe de formule -S(O)R^{L1}, R^{L1} représentant un groupe alkyle, un groupe halogénoalkyle, un groupe alcanoylaminoalkyle, un groupe alcénoylaminoalkyle, un groupe aryle ou un groupe hétérocyclique aromatique, et R^{3p} représente un groupe protecteur de carboxy) avec un dérivé de pyrrolidine de formule (III) : 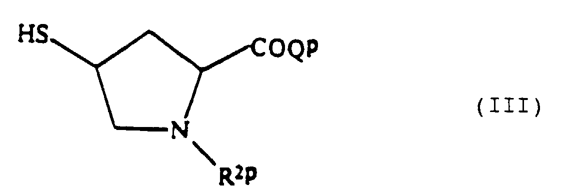 (dans laquelle R^{2p} représente l'un quelconque des groupes ou atomes représentés par R² ou ce quelconque groupe ou atome qui a été protégé, et Q^{p} représente l'un quelconque des groupes ou atomes représentés par Q ou ce quelconque groupe ou atome qui a été protégé et, dans le cas où le groupe Q^{p} contient un atome d'azote quaternaire, le composé est accompagné par un anion assurant l'électroneutralité).
et, si nécessaire, à enlever l'un quelconque ou plusieurs des groupes protecteurs,
et, de façon facultative, à salifier et/ou estérifier le produit.
